# FASCICULE DE BREVET EUROPEEN

(11) **EP 0 839 059 B1**
(45) Date de publication et mention de la délivrance du brevet: **17.04.2002**
(21) Numéro de dépôt: 96925791.4
(22) Date de dépôt: 16.07.1996
(51) Int. Cl.: A61K 51/04

(54) **PRODUITS RADIOPHARMACEUTIQUES A TROPISME CARDIAQUE COMPORTANT UN COMPLEXE NITRURO D'UN METAL DE TRANSITION ET AYANT UNE CLAIRANCE MYOCARDIQUE RAPIDE**
CARDIOTROPISCHE RADIOPHARMAZEUTIKA,DIE EINEN ÜBERGANGSMETALL-NITRID-KOMPLEX ENTHALTEN UND DIE EINE RASCHE MYOCARDIAL-ELIMINIERUNG AUFWEISEN
CARDIOTROPIC RADIOPHARMACEUTICALS COMPRISING A TRANSITION METAL NITRIDE COMPLEX AND HAVING RAPID MYOCARDIAL CLEARANCE

(30) Priorité: 17.07.1995 FR 9508606
(43) Date de publication de la demande: 06.05.1998
(73) Titulaire: CIS BIO INTERNATIONAL, 91190 Gif-sur-Yvette (FR)
(72) Inventeur: BELLANDE, Emmanuel, F-91160 Saulx-les-Chartreux (FR); LAINE, Jacques, F-92160 Antony (FR); COMAZZI, Véronique, F-92170 Vanves (FR); PASQUALINI, Roberto, F-92140 Clamart (FR)
(74) Mandataire: Des Termes, Monique
(86) Numéro de dépôt international: FR9601103
(87) Numéro de publication internationale: WO9703705

(56) Documents cités:
- WO-A-89/08657
- WO-A-90/06137
- WO-A-92/10214
- WO-A-93/01839
- FR-A- 2 698 272
- NUCLEAR MEDICINE AND BIOLOGY, vol. 21, no. 2, 1 Février 1994, pages 263-268, XP000434379 COULAIS Y ET AL: "SYNTHESIS, CHARACTERIZATION AND BIODISTRIBUTION OF NEW 99MTC OXO AND NITRIDO COMPLEXES WITH BI- AND TETRADENDATE UNSATURATED NS AND N2S2 SCHIFF BASES DERIVED FROM 2-AMINOCYCLOPENTENE-1- DITHIOCARBOXYLIC ACID AS POTENTIAL HEART IMAGING AGENTS"
- DATABASE CHEMABS CHEMICAL ABSTRACTS SERVICE, COLUMBUS, OHIO, US AN=123:137631, GHEZZI, CATHERINE ET AL: "Myocardial kinetics of TcN-NOET: a neutral lipophilic complex tracer of regional myocardial blood flow" XP002001329 & J. NUCL. MED. (1995), 36(6), 1069-77 CODEN: JNMEAQ;ISSN: 0161-5505, 1995,

## Description

La présente invention a pour objet un produit radiopharmaceutique à tropisme cardiaque, qui comprend un complexe nitruro d'un métal de transition M comportant une partie centrale M ≡ N, utilisable pour la scintigraphie myocardique.

Selon l'invention, on entend par métal de transition un métal dont la couche d est partiellement remplie dans le degré d'oxydation usuel de ce métal. Il s'agit des éléments remplissant les périodes III à XII du tableau périodique des éléments à dix-huit colonnes.

A titre d'exemple de tels métaux, on peut citer Tc, Ru, Co, Pt, Fe, Os, Ir, W, Re, Cr, Mo, Mn, Ni, Rh, Pd, Nb et Ta.

Des complexes nitruro de technétium ont été décrits par J. Baldas et Col. dans les documents suivants : demande de brevet internationale WO-85/03 063, J. Chem. Soc. Dalton Trans., 1981, pages 1798-1801; et le livre "Technetium in Chemistry and Nuclear edicine", Ed. M. Nicolini, G. Bandoli, U. Mazzi, Cortine Int. Verone, 1986, pages 103 à 108.

Des complexes nitruro de technétium ayant un tropisme cardiaque ont été décrits dans le document WO-A-90/06137. Ces complexes comportent une partie centrale M ≡ N et des ligands du type dithiocarbamato ayant des groupes latéraux du type alkyle, éventuellement substitués par des groupements alkoxy, alkylcarboxy, carbamoyle ou amino.

Ces complexes ont de bonnes propriétés biologiques en ce qui concerne leur captation cardiaque, mais ils peuvent présenter une fixation pulmonaire précoce gênante pour les examens myocardiques, et ils sont éliminés de l'organisme, en particulier du coeur, assez lentement.

Ceci constitue un inconvénient pour l'examen scintigraphique du myocarde. En effet, un examen complet du myocarde nécessite généréralement deux injections du produit radiopharmaceutique, l'une au repos et l'autre après un test d'effort. Avec les produits radiopharmaceutiques qui montrent une fixation stable dans le myocarde, ces deux injections doivent être espacées au minimum de 4 heures afin d'éliminer l'activité cardiaque résiduelle due à la première injection. Il serait donc d'un grand intérêt de disposer de produits radiopharmaceutiques à tropisme cardiaque éliminés plus rapidement.

Un autre problème que l'on rencontre avec des produits radiopharmaceutiques de ce type est leur fixation hépatique qui peut être gênante si elle est persistante. En effet, une telle fixation ne permet pas de réaliser un examen scintigraphique satisfaisant du myocarde lorsque la seconde injection est trop proche de la première. Il serait donc intéressant de disposer de produits radiopharmaceutiques à tropisme cardiaque éliminés rapidement du foie et du coeur.

La présente invention a précisément pour objet des produits radiopharmaceutiques comprenant un complexe nitruro de métal de transition, qui sont éliminés plus rapidement du coeur et ne présentent pas de fixations pulmonaire et hépatique gênantes. Ces complexes comportent également des ligands du type dithiocarbamate mais les groupes alkyle de ces ligands sont soit une chaîne ramifiée comportant au moins une fonction éther, soit une chaîne ramifiée dont un atome de carbone est substitué par deux groupes alkoxy, ce qui correspond à une fonction acétal.

Selon l'invention, le produit radiopharmaceutique comprend un complexe d'un métal de transition répondant à la formule :

(M ≡ N) L¹L² (I)

dans laquelle M est un métal de transition, et L¹ et L² qui peuvent être identiques ou différents, répondent à la formule : 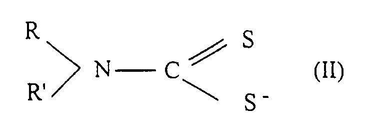 dans laquelle R et R' qui sont identiques ou différents, représentent :
- un groupe alkyle ;
- un groupe de formule : 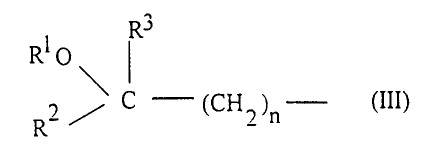 dans laquelle :
   R¹ est un groupe alkyle, R² est un groupe alkyle ou un groupe alcoxy ou R¹ et R² forment ensemble le groupe ―CH₂CH₂0― ;
   R³ est un atome d'hydrogène ou un groupe alkyle, et n est égal à 0, 1, 2 ou 3 ;
- un groupe de formule : 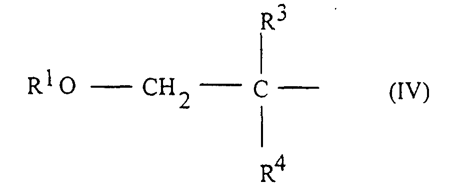 dans laquelle R¹ et R³ sont tels que définis ci-dessus et R⁴ est un groupe alkyle,
- le groupe de formule : 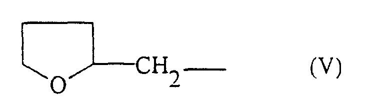
- un groupe de formule :

   R¹O(CH₂CH₂O)ₚ CH₂CH₂- (VI)
dans laquelle R¹ est tel que défini ci-dessus, et p est égal à 1 ou 2, ou
dans laquelle R et R' forment ensemble avec l'atome d'azote auquel ils sont liés le groupe de formule : 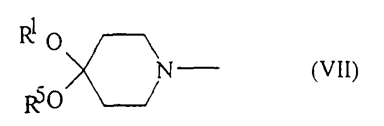 dans laquelle R¹ et R⁵ sont des groupes alkyle identiques ou différents, ou dans laquelle R¹ et R⁵ forment ensemble -CH₂-CH₂-, à condition que R et R' ne représentent pas tous deux un groupe alkyle.

Les ligands L¹ et L² du complexe de métal de transition de l'invention comportent donc un groupe alkyle ramifié de formule (III) ou de formule (IV), un groupe tétrahydrofurfuryle, un groupe du type polyéther linéaire, ou un groupe du type dioxaspiro ou dialkoxy pipéridino, qui leur confére de meilleures propriétés de clairance myocardique.

Dans les complexes de l'invention, le métal de transition M est choisi en particulier en fonction de l'application du produit radiopharmaceutique.

Ainsi, lorsqu'on veut utiliser le produit pour le diagnostic, on utilise un métal de transition radioactif, ayant une période relativement courte, par exemple le technétium-99m.

Dans le cas où l'on veut utiliser le produit radiopharmaceutique pour la thérapie, on utilise un métal de transition émettant un rayonnment efficace pourla thérapie, et ayant une durée de vie plus longue, tel que le rhénium, par exemple Re-186 ou Re-188.

Dans les formules données ci-dessus pour R et R', les groupes alkyle représentés par R¹, R², R³, R⁴ et R⁵ peuvent être linéaires ou ramifiés. Généralement on utilise des groupes alkyle de 1 à 5 atomes de carbone, en particulier les groupes méthyle et éthyle.

Le groupe alkyle utilisé pour R ou R' peut être également linéaire ou ramifié ; de préférence, il comprend 1 à 5 atomes de carbone.

Lorsque R² représente un groupe alkoxy, il peut s'agir également d'un groupe linéaire ou ramifié ; de préférence, ce groupe alkoxy comprend de 1 à 5 atomes de carbone.

Dans le complexe de métal de transition de l'invention, les ligands L¹ et L² peuvent être identiques ou différents. De préférence, ils sont identiques.

Selon un premier mode de réalisation de l'invention, l'un au moins des L¹ et L² répond à la formule (II) dans laquelle :
- R et/ou R' représentent un groupe acétal de formule : 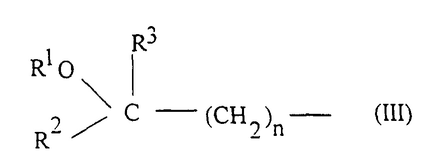
dans laquelle R¹ représente un groupe alkyle, par exemple le groupe méthyle ou éthyle, R² représente un groupe alcoxy, par exemple le groupe méthoxy ou éthoxy, R³ représente un atome d'hydrogène ou un groupe alkyle, par exemple le groupe méthyle, et n est égal à 0, 1, 2 ou 3.

Selon un second mode de réalisation de l'invention l'un au moins des L¹ et L² répond à la formule (II) dans laquelle R et/ou R'représentent un groupe de formule : 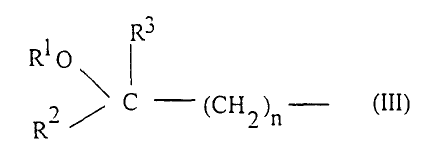 dans laquelle R¹, R² et R³ sont des groupes alkyle, identiques ou différents, par exemple le groupe méthyle.

Selon un troisième mode de réalisation de l'invention, l'un au moins des L¹ et L² répond à la formule (II) dans laquelle :
- R et/ou R'répondent à la formule (IV) : 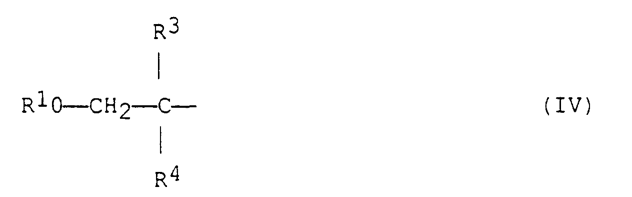
dans laquelle R¹ représente un groupe alkyle, par exemple le groupe méthyle, R³ représente un atome d'hydrogène et R⁴ représente un groupe alkyle tel que le groupe méthyle ou éthyle.

Selon un quatrième mode de réalisation de l'invention, l'un au moins des L¹ et L² répond à la formule (II) dans laquelle :
- R et/ou R'représentent le groupe de formule :

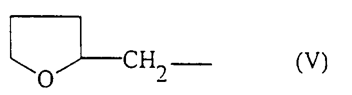

Les complexes nitruro de technétium utilisés dans l'invention peuvent être préparés par des procédés classiques, par exemple par le procédé de Baldas. Toutefois, on préfère généralement les préparer par des procédés plus simples, faciles à mettre en oeuvre dans un service hospitalier et conduisant à des rendements élevés, par exemple par les procédés décrits dans WO-A-93 01839, WO-A-92/00982 et WO-A-90/06137.

De préférence, selon l'invention on utilise un le procédé décrit dans WO92/00982 qui consiste à faire réagir en solution un composé oxygéné du métal de transition M, avec
- 1) un ligand azoté constitué soit par un azoture d'ammonium ou de métal pharmaceutiquement acceptable, soit par un composé azoté comportant un motif N-N dans lequel les N sont reliés à des atomes d'hydrogènes et/ou à des groupements organiques monovalents, par exemple par l'intermédiaire d'un atome de carbone ou d'un atome de S, ou dans lequel l'un des N est relié à l'atome de carbone d'un groupement organique bivalent par l'intermédiaire d'une double liaison et l'autre N est relié à des atomes d'hydrogène et/ou à des groupements organiques monovalents, par exemple par l'intermédiaire d'un atome de carbone ;
- 2) un agent réducteur constitué soit par du dithionite d'ammonium ou de métal pharmaceutiquement acceptable, soit par de l'étain II présent sous forme ionique dans la solution, et
- 3) un composé répondant à la formule : 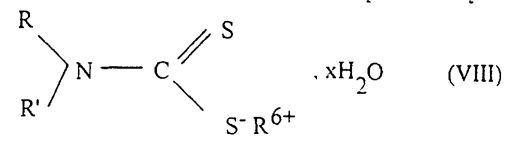 dans laquelle R et R' ont les significations données ci-dessus, R⁶ est un ion de métal alcalin, H⁺ ou NH⁴⁺, et x est égal à 0 ou est un nombre entier allant de 1 à 5.

Cette réaction entre le composé oxygéné du métal de transition, l'agent réducteur, le ligand azoté et le composé de formule (VIII) peut être effectué en une seule étape ou en deux étapes.

De préférence, on opère en deux étapes. Ainsi, dans une première étape on fait réagir le composé oxygéné du métal de transition avec le ligand azoté et l'agent réducteur, et dans une deuxième étape, on fait réagir le produit obtenu à la suite de la première étape avec le composé de formule (VIII).

Lorsqu'on utilise comme agent réducteur l'étain (II), on peut l'introduire dans la solution à partir d'un ou plusieurs réactifs capables de le maintenir sous forme ionique en présence du ligand azoté et du composé de formule (VIII).

On peut en particulier introduire l'étain sous la forme de sel d'étain (II), par exemple de chlorure stanneux dihydraté. Pour le maintenir sous forme ionique dans la solution, on ajoute simultanément un agent complexant ayant un pouvoir complexant vis-à-vis de l'étain plus fort que ceux du ligand azoté et du composé de formule (VIII).

L'agent complexant peut être en particulier l'acide 1,2-diaminopropane-N,N,N',N'-tétracétique ou un sel de celui-ci.

De préférence, selon l'invention, on utilise comme agent réducteur le chlorure stanneux dihydraté, et comme ligand azoté le succinyl dihydrazide.

Les composés de formule (VIII) utilisés pour la préparation des produits radiopharmaceutiques de l'invention peuvent être préparés par des procédés classiques, à partir des amines correspondantes de formule : 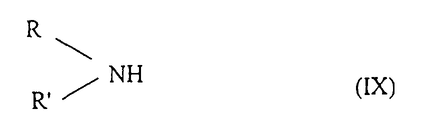 dans laquelle R et R' sont tels que définis ci-dessus, par réaction de ces amines avec du disulfure de carbone en présence de soude.

Les amines secondaires de formules (IX) peuvent être préparées par réaction d'une amine primaire de formule :

R-NH₂

avec un halogène de formule R'X dans laquelle X est un halogène, de préférence Br.

Le produit radiopharmaceutique de l'invention est généralement préparé au moment de l'utilisation. Aussi, l'invention a également pour objet une trousse pour la préparation d'un produit radiopharmaceutique à tropisme cardiaque, qui comprend :
- un premier flacon contenant un sel d'étain et un agent complexant capable de maintenir l'étain sous forme ionique, et
- un deuxième flacon contenant le ligand azoté, et
- un troisième flacon contenant un composé répondant à la formule (VIII).

Selon une variante de réalisation, la trousse ne comprend que deux flacons,
- un premier flacon contenant le sel d'étain, l'agent complexant pour maintenir l'étain sous forme ionique et le ligand azoté, et
- un deuxième flacon contenant le composé de formule (VIII).

Avantageusement, le composé de formule (VIII) est choisi parmi
- le (N-éthyl, N-(R,S)-2-méthoxy isopropyl) dithiocarbamate de sodium,
- le(N-éthyl, N(R)-2-méthoxy isopropyl) dithiocarbamate de sodium,
- le (N-éthyl, N-(S)-2 méthoxy isopropyl) dithiocarbamate de sodium,
- le [N,N-bis(2,2-diméthoxy éthyl)] dithiocarbamate de sodium,
- le [N-(2,2-diméthoxyéthyl), N-(3,3-diméthoxypropyl)] dithiocarbamate de sodium,
- le [N-(2,2-diméthoxyéthyl), N-éthyl)] dithiocarbamate de sodium.

Les produits présents dans chaque flacon peuvent être sous forme de solution ou sous forme lyophilisée.

Avec cette trousse, on peut préparer directement le produit radiopharmaceutique voulu, dans un service hospitalier de médecine nucléaire, en ajoutant au contenu du premier flacon une solution du composé oxygéné du métal de transition, par exemple une solution de pertechnétate de métal acalin ou d'ammonium, puis en ajoutant au produit ainsi obtenu le contenu du deuxième flacon.

Etant donné que les produits sont destinés à une injection intraveineuse à des être vivants, on doit utiliser des conditions appropriées de fabrication et de mise en oeuvre pour obtenir des solutions convenablement stériles et apyrogènes.

Pour préparer les solutions, on peut utiliser de l'eau stérile et apyrogéne ou des solutions alcooliques ou hydroalcooliques, stériles et apyrogènes, et effectuer un stockage des solutions sous azote.

Pour préparer des compositions lyophilisées, on soumet à une lyophilisation dans un équipement classsique des solutions obtenues dans les mêmes conditions que précédemment.

Les produits radiopharmaceutiques de l'invention peuvent être utilisés en particulier pour la scintigraphie du myocarde.

Dans ce cas, après préparation du complexe nitruro de technétium, on injecte celui-ci au patient à examiner, et on procède ensuite à un examen du coeur par scintigraphie.

Pour l'injection du produit, les quantités des différents ligands sont telles qu'elles correspondent sensiblement à la stoechiométrie des complexes à obtenir. La quantité finale injectée dépend en particulier des ligands utilisés et de leur toxicité.

Généralement, on obtient des résultats satisfaisants en utilisant des quantités totales de ligands allant de 0,05 à 0,40 mg/kg de poids corporel.

La dose totale de métal de transition, par exemple de technétium, se situe généralement dans la gamme de 185 à 740 Mbq (5 à 20 millicuries).

Après l'administration du complexe nitruro de métal de transition, on peut effectuer un examen satisfaisant dans un délai très court, par exemple de 2 à 5 minutes aprés injection, en obtenant un bon contraste, des images nettes et une bonne détection des lésions.

De plus, on peut effectuer avec ces composés deux examens successifs en un temps très bref, par exemple en réalisant la seconde injection une heure après la première, en raison de la clairance rapide de ces produits radiopharmaceutiques.

En effet, les produits radiopharmaceutiques de l'invention, notamment ceux dans lesquels les ligands comportent un groupe R ou R' ramifié à substituant alcoxy, répondant à la formule (III) avec R² et R³ étant des groupes alkyle ou avec R² étant un groupe alcoxy et R³ étant un atome d'hydrogène, présentent l'avantage d'être éliminés du foie par voie biliaire et de concentrer ainsi l'activité résiduelle dans la vésicule biliaire. Ainsi, même en réalisant la seconde injection 1 heure après la première, on n'est pas gêné par l'activité hépatique résiduelle due à la première injection puisque cette activité résiduelle se trouve dans la vésicule biliaire, soit dans une zone qui n'interfère pas avec la zone d'examen du coeur.

Il n'en serait pas de même si on utilisait des produits radiopharmaceutiques tels que ceux de l'art antérieur dans lesquels les ligands comportent un groupe R de type alcoxy ou alcoxyalkyle linéaire comme les composés des exemples 11 et 17 du document WO-A-90 06137 mentionné précédemment.

En effet, dans le cas de ces composés où les groupes R des ligands L¹ et L² répondent respectivement aux formules : 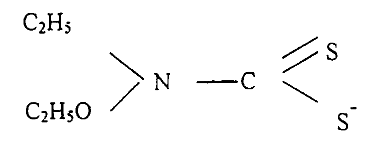 et 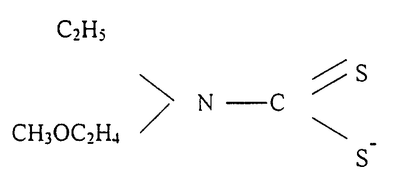 La fixation hépatique résiduelle concerne tout le foie, 1 heure après l'injection, car il n'y a pas de concentration d'activité dans la vésicule biliaire.

D'autres caractéristiques et avantages de l'invention apparaîtront mieux à la lecture des exemples suivants donnés bien-entendu à titre illustratif et non limitatif.

### EXEMPLE 1 : Préparation du complexe nitruro-bis[N-éthyl, N-(R,S,2-méthoxy isopropyl) dithiocarbamato] ^{99m} Tc(V).

### 1) Préparation du ligand (N-éthyl,N-(R,S)-2-méthoxy isopropyl)dithiocarbamate de sodium.

### a) Préparation de la (N-éthyl,N-(R,S)-2-méthoxy isopropyl)amine.

On dissout 0,1 mol de (R,S)-2-méthoxy isopropyl amine (Aldrich) dans 100 ml d'eau contenant 0,2 mol de NaOH. On ajoute goutte à goutte 0,1 mol de bromoéthane. La solution est portée au reflux pendant une nuit. La phase aqueuse est extraite par 200 ml d'éther diéthylique. La phase organique est lavée trois fois avec 20 ml de NaOH 1N dans de l'eau afin d'éliminer l'amine primaire n'ayant pas réagi. La phase organique est séchée sur sulfate de sodium et l'on distille l'amine secondaire TE_{b} = 121°C sous 760 mm Hg.

### b) Préparation de (N-éthyl,N-(R,S)-2-méthoxy isopropyl) dithiocarbamate de sodium.

On dissout 0,05 mol de l'amine préparée en a) dans 200 ml d'éther contenant 0,1 mol de NaOH en pastilles. On ajoute goutte à goutte 0,1 mol de CS₂ et on laisse réagir 4 heures à la température ambiante. La phase organique est filtrée et l'on ajoute 500 ml de n-heptane. On laisse recristalliser une nuit à 4°C. et l'on obtient un solide blanc (dithiocarbamate de sodium donné en titre) qui est recristallisé dans un mélange éther-n-heptane.
Analyse RMN dans le diméthyl sulfoxyde deutéré (DMSO-D6) 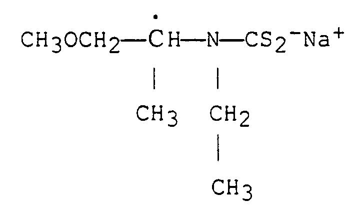
CH₃O : singulet 3,35 ppm
CH₃O-CH₂ : doublet 4,05 ppm 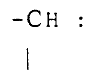
quintuplet 6,3 ppm
CH₃-CH : doublet 0,9 ppm
CH₃-CH₂-N triplet 1,1 ppm
CH₃-CH₂-N quadruplet 3,65 ppm.

### 2) Préparation de l'intermédiaire (Tc≡N)²⁺

On ajoute 0,5 à 5 ml d'une solution de pertech nétate de sodium (^{99m} TcO₄⁻),37KBQ à 3,7 GBQ, à un flacon de type pénicilline contenant sous forme lyophilisée :
- 20 µg de SnCl₂,2H₂O (chlorure stanneux dihydraté)
- 10 mg de succinyl dihydrazide (SDH),
- 5 mg d'acide 1,2-diaminopropane-N,N,N',N'-tétraacétique.
- 10 mg de γ-cyclodextrine,
- 0,05 mmol de tampon phosphate à pH = 7,80. On laisse réagir 10 minutes à la température ambiante et l'on obtient l'intermédiaire (Tc ≡ N)²⁺

### 3) Préparation du complexe final.

On ajoute 1 ml d'une solution de (N-éthyl, N (RS)2-méthoxy isopropyl) dithiocarbamate de sodium préparé en 1), à 10 mg/ml dans l'eau au flacon contenant l'intermédiaire (Tc ≡ N)²⁺. On laisse réagir 5 minutes à la température ambiante et l'on obtient le complexe final.

La pureté radiochimique est testée en effectuant une chromatographie sur couche mince : phase inverse (Whatman KC18F, éluant Methanol 30 %, acétonitrile 30 %, THF 20 %, AcNH₄ 0,5 M dans H₂O 20 %). Le radiopharmaceutique obtenu a un Rf = 0,52. la pureté radiochimique est supérieure ou égale à 96 %.

### EXEMPLE 2 : Préparation du complexe nitruro-bis [(N-éthyl Ne (R)-2 méthoxy isopropyl) dithiocarbamato] ^{99m}Tc(V)

### 1) Synthèse du ligand (N-éthyl, N((R)-2 méthoxy isopropyl) dithiocarbamate de sodium.

On prépare tout d'abord la R-1-méthoxy isopropylamine de la façon suivante.

On dissout 0,2 mol de R-2-amino-1-propanol (Aldrich) dans 200 ml d'éther anhydre sous atmosphère d'azote. On ajoute 0,25 mol de sodium métal (Na). On laisse réagir au reflux pendant 6 heures. On ajoute ensuite goutte à goutte 0,2 mol d'iodure de méthyle. La solution est filtrée, le filtrat est séché sur sulfate de sodium et l'on distille l'amine. TE_{b} = 93°C à pression atmosphérique.

On prépare ensuite l'amine substituée puis le dithiocarbamate de sodium correspondant en suivant le même mode opératoire que dans l'exemple 1, étapes 1a) et 1b).

On obtient ainsi le ligand N-éthyl, N(-2(R) méthoxy isopropyl)dithiocarbamate de sodium. L'analyse de ce produit par RMN dans le DMSO conduit à un spectre identique à celui obtenu dans l'exemple 1.

### 2) Préparation de l'intermédiaire (Tc≡N)²⁺

Celui-ci est préparé en suivant le même mode opératoire que dans l'exemple 1.

### 3) Préparation du complexe final.

Celui-ci est préparé comme dans l'exemple 1 à partir de l'intermédiaire obtenu précédemment mais en utilisant le dithiocarbamate de sodium préparé dans l'étape 1). Il présente les caractéristiques suivantes :
Rf = 0,52
Pureté radiochimique ≥ 97 %

### EXEMPLE 3 à 6 :

On suit le même mode opératoire que dans les exemples 1 et 2 pour préparer les complexes de technétium donnés dans le tableau 1, les caractéristiques de ces complexes sont également données dans ce tableau.

### EXEMPLE 7 : Préparation du complexe nitruro-bis[N-éthyl, N-(R,S)-2 méthoxy propyl)dithiocarbamato] ^{99m}Tc(V)

On prépare tout d'abord le ligand N-éthyl, N-(R,S-2 méthoxy propyl)dithiocarbamate de sodium en opérant de la façon suivante.

### a) Préparation de la R,S-2 méthoxy propyl amine.

On dissout 0,2 mol de (R,S)-1-amino-2 propanol (Aldrich) dans 200 ml d'éther anhydre sous atmosphère d'azote. On ajoute 0,25 mol de sodium métal. On laisse réagir au reflux pendant 6 heures. On ajoute ensuite goutte à goutte 0,2 mol d'iodure de méthyle. La solution est filtrée, le filtrat est séché sur sulfate de sodium et l'on distille l'amine. TE_{b} = 106°C sous 760 mmHg.

### b) Préparation du ligand

On procéde ensuite comme dans l'exemple 1 pour préparer le ligand à partir de cette amine.

On suit ensuite le même mode opératoire que dans l'exemple 1 pour préparer le complexe de technétium en partant de ce ligand.

Les caractéristiques de ce complexe sont données dans le tableau 1.

### EXEMPLE 8 :

On suit le même mode opératoire que dans l'exemple 1 pour préparer le complexe de technétium mentionné dans le tableau 1 à partir de la R,S-1-(méthoxyméthyl)propyl amine que l'on prépare de la façon suivante.

On dissout 0,2 mol de (R,S)-2-amino-1-butanol (Aldrich) dans 200 ml d'éther anhydre sous atmosphère d'azote. On ajoute 0,25 mol de sodium métal. On laisse réagir au reflux pendant 6 heures. On ajoute ensuite goutte à goutte 0,2 mol d'iodure de méthyle. La solution est filtrée, le filtrat séché sur sulfate de sodium et l'on distille l'amine TE_{b} = 102°C sous 760 mm Hg.

Les caractéristiques du complexe obtenu sont données dans le tableau 1.

### EXEMPLE 9 :

On suit le même mode opératoire que dans les exemples précédents pour préparer le complexe de technétium mentionné dans le tableau 1, à partir de la 2-méthoxyisobutylamine que l'on prépare de la façon suivante.

On dissout 0,3 mol de 2,2-diméthyl aziridine dans 80 ml de méthanol contenant 0,35 mol de complexe méthanol trifluoroborane. On laisse réagir 7 jours à température ambiante et l'on ajoute 0,45 mol de méthylate de sodium. La solution est filtrée et distillée. On sépare les deux amines obtenues :
- 2 méthoxy isobutylamine. TE_{b} = 126°C sous 760 mmHg
- 2 méthoxy tert-butylamine. TE_{b} = 103°C sous 760 mmHg

Les caractéristiques du complexe obtenu sont données dans le tableau 1.

### EXEMPLE 10 :

On suit le même mode opératoire que dans l'exemple 1 pour préparer le complexe de technétium mentionné dans le tableau 1 à partir de la 2-méthoxytert-butylamine isolée dans l'exemple précédent.

Les caractéristiques du complexe obtenu sont données dans le tableau 1.

### EXEMPLES 11 et 12 :

On prépare les complexes de technétium mentionnés dans le tableau 1, en suivant le même mode opératoire que dans l'exemple 1 mais en partant de la tétrahydrofurfurylamine que l'on traite par du bromométhane dans l'exemple 11 et par du bromoéthane dans l'exemple 12.

Les caractéristiques des complexes obtenus sont données dans le tableau 1.

### EXEMPLE 13 :

On prépare le complexe mentionné dans le tableau 1 en suivant le même mode opératoire que dans l'exemple 1, à partir de la N-éthyl, N-2-(2-méthoxy éthoxy) éthylamine que l'on prépare de la façon suivante.

On ajoute goutte à goutte 0,2 mol de 1-bromo-2-(2-méthoxy éthoxy) éthane (Aldrich) à une solution aqueuse d'éthylamine. On laisse réagir 6 heures à reflux. On filtre la solution qui est extraite avec de l'éther. La phase organique est distillée.

Les caractéristiques du complexe obtenu sont données dans le tableau 1.

### EXEMPLE 14 :

On prépare le complexe mentionné dans le tableau 1 en suivant le même mode opératoire que l'exemple 1, à partir du ligand N,N-bis(2,2-diméthoxyéthyl)dithiocarbamate de sodium qui est préparé à partir de la N,N-bis(2,2-diméthoxyéthyl)amine obtenue de la façon suivante.

On dissout 0,2 mol d'aminoacétaldéhyde diméthyl acétal (Aldrich) dans 100 ml d'eau contenant 0,3 mol de NaOH. On ajoute goutte à goutte 0,2 mol de bromoacétaldéhyde diméthyl acétal (Aldrich). La solution est portée à reflux pendant une nuit. La phase aqueuse est extraite par 200 ml d'éther diéthylique. La phase organique est lavée trois fois avec 20 ml de NaOH 1N dans l'eau afin d'éliminer l'amine primaire n'ayant pas réagi. La phase organique est séchée sur sulfate de sodium et l'on distille l'amine secondaire : E = 70°C sous 2 mm Hg.

L'analyse par RMN dans le DMSO deutéré du ligand obtenu 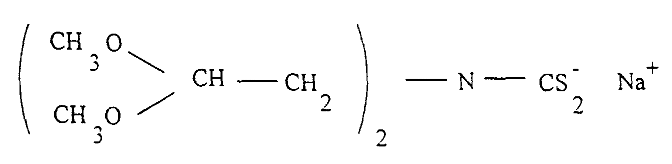 donne les résultats suivants :
CH₃O : singulet 3,4 ppm
CH : triplet 4,9 ppm
CH₂ : doublet 4,1 ppm

Les caractéristiques du complexe obtenu sont données dans le tableau 1.

### EXEMPLES 15 à 24:

On suit le même mode opératoire que dans les exemples précédents pour préparer les complexes mentionnés dans le tableau 1, en partant des amines et des halogénures correspondant aux ligands dû complexe.

Les caractéristique des complexes obtenus sont données dans le tableau 1.

### EXEMPLE 25

Les propriétés biologiques des complexes obtenus dans les exemples 1 à 24 sont évaluées en déterminant la captation cardiaque chez des singes babouins pesant entre 9 et 12 kg.

Dans ce but, on injecte 0,5 ml de solution du complexe final correspondant à une activité de 74 MBQ. L'animal est anesthésié par un mélange kétamine/valium et placé sous une gamma caméra. La captation de la radioactivité par le coeur et par les organes environnants (poumons, foie) est déterminée par une acquisition dynamique pendant 60 minutes après l'injection. On détermine des zones d'intérêt pour chaque organe et l'on calcule les valeurs de captation exprimées en coups par minute par pixel (unité de surface) et par mCi (cpm.pix⁻¹mCi⁻¹). Ces valeurs sont corrigées de la décroissance radioactive du radioélément.

Des prélèvements sanguins sont effectués au cours de l'examen et leur activité est comptée, ce qui permet de calculer le pourcentage de radioactivité présent dans le sang total (activité circulante).

Les valeurs de captation cardiaque, les rapports coeur/poumons et coeur/foie donnant un indice du contraste scintigraphique et les valeurs d'activité sanguine, obtenues avec les complexes des exemples 1 à 24, sont reportées dans le tableau 2 qui suit.

Dans ce tableau, on a donné à titre comparatif les résultats obtenus dans les mêmes conditions avec le traceur cardiaque MIBI commercialisé par Dupont et avec le complexe de l'art antérieur ^{99m}TcN-NOET, qui est le nitruro-bis[N-éthoxy, N-éthyl-dithiocarbamato]^{99m}Tc (V) et qui répond à la formule (I) dans laquelle L¹ et L² sont :

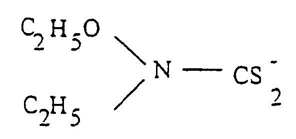

Les résultats du tableau 2 montrent que la plupart des complexes ont une captation cardiaque supérieure à celle du MIBI et équivalente à celle de ^{99m}TcN-NOET.

Par contre l'activité cardiaque diminue au cours du temps, ce qui se traduit par une baisse des rapports scintigraphiques coeur/poumons et coeur/foie.

La clairance sanguine est rapide pour la plupart des complexes testés.

La période idéale pour l'imagerie cardiaque avec ce type de complexe se situe donc entre 5 et 30 minutes après l'injection.

L'élimination rapide du myocarde peut permettre une deuxième injection 1 heure après la première injection, car l'activité hépatique résiduelle n'est pas gênante puisqu'elle se concentre dans la vésicule biliaire avec ce type de traceur.

En effet, les images scintigraphiques montrent que 1 heure après l'injection, l'activité hépatique résiduelle est concentrée dans la vésicule biliaire sauf pour le complexe de l'exemple 24.

En revanche, des images scintigraphiques prises chez le singe avec les composés des exemples 11 et 17 du document WO-A-90/06137 ont montré que l'activité hépatique résiduelle se maintient dans la totalité du foie.

On remarque par ailleurs que les complexes les plus intéressants sont ceux des exemples 1, 2, 3, 7, 12, 14, 15 et 18, les meilleurs étant ceux des exemples 1, 2, 3, 14, 15 et 18.

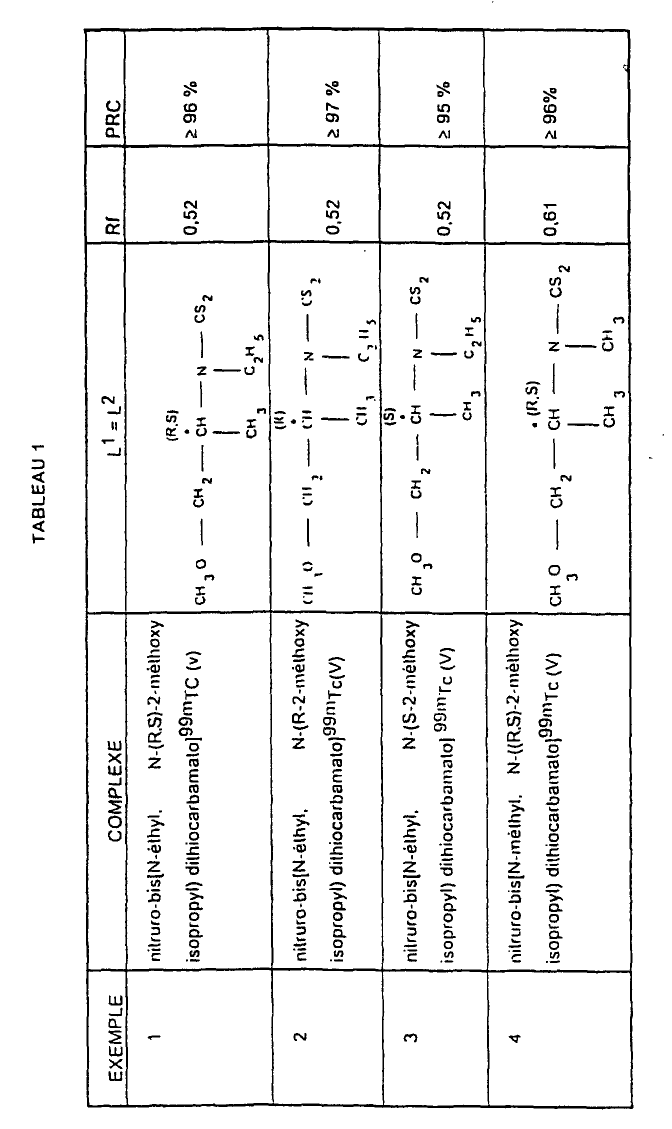

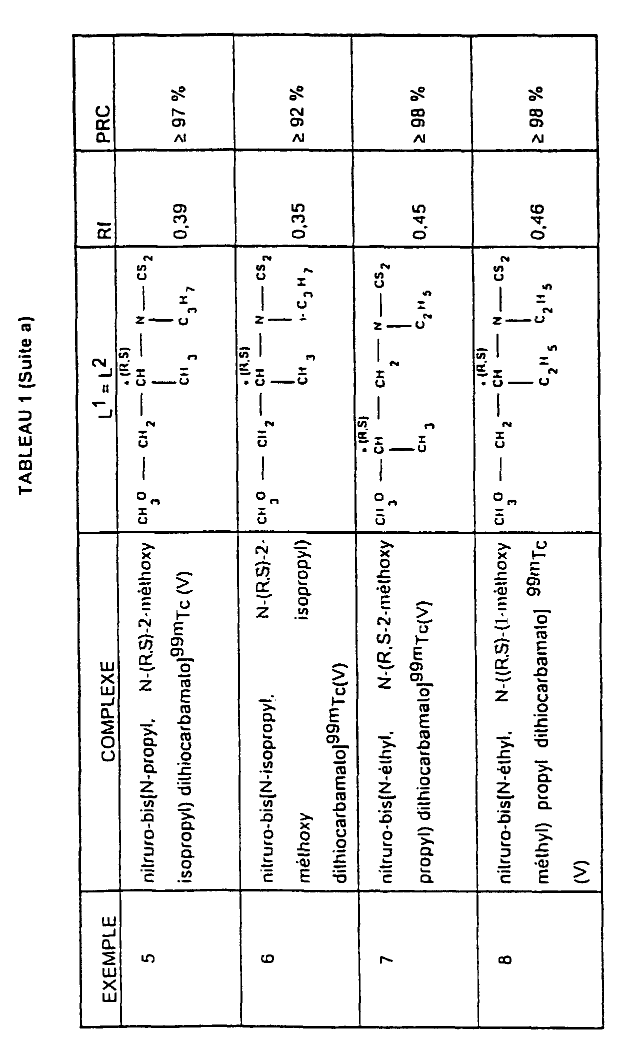

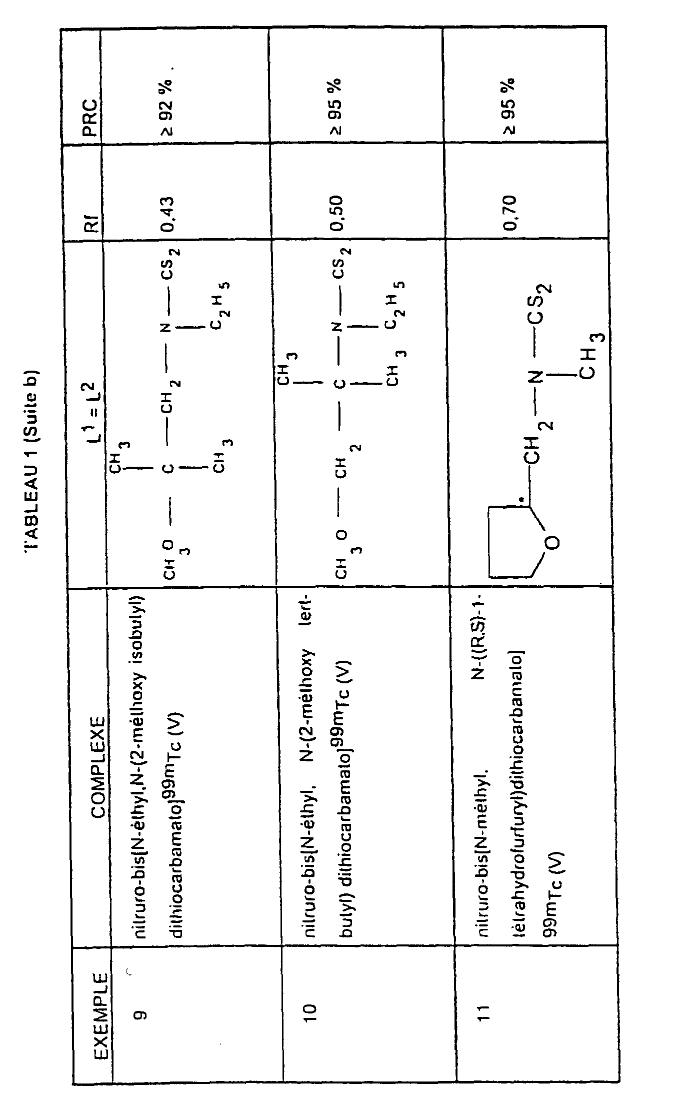

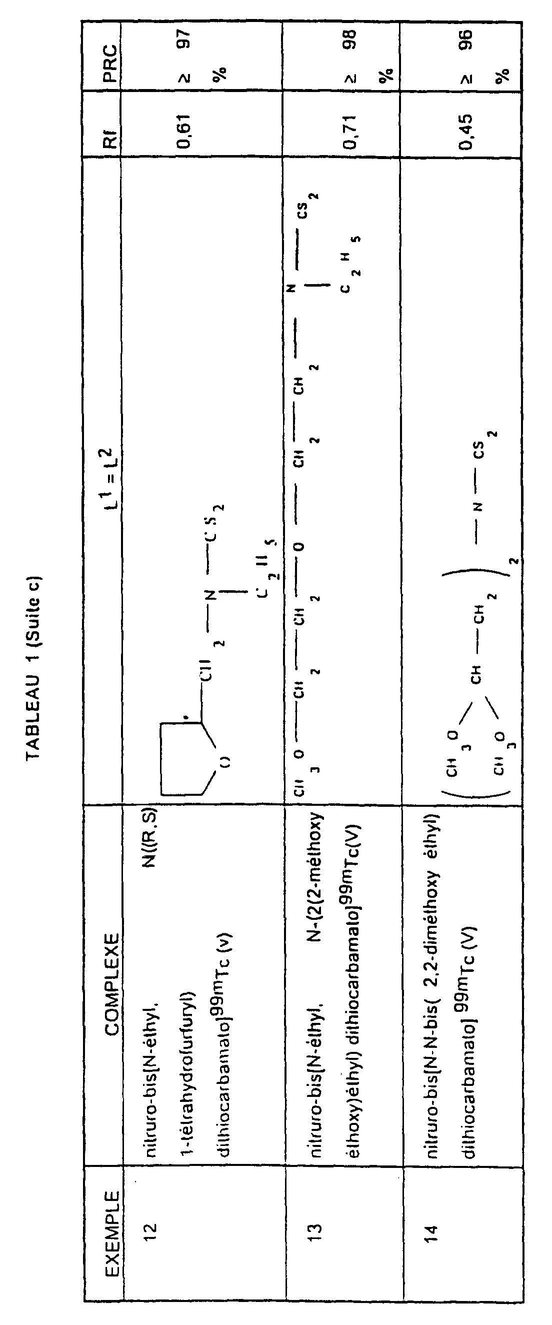

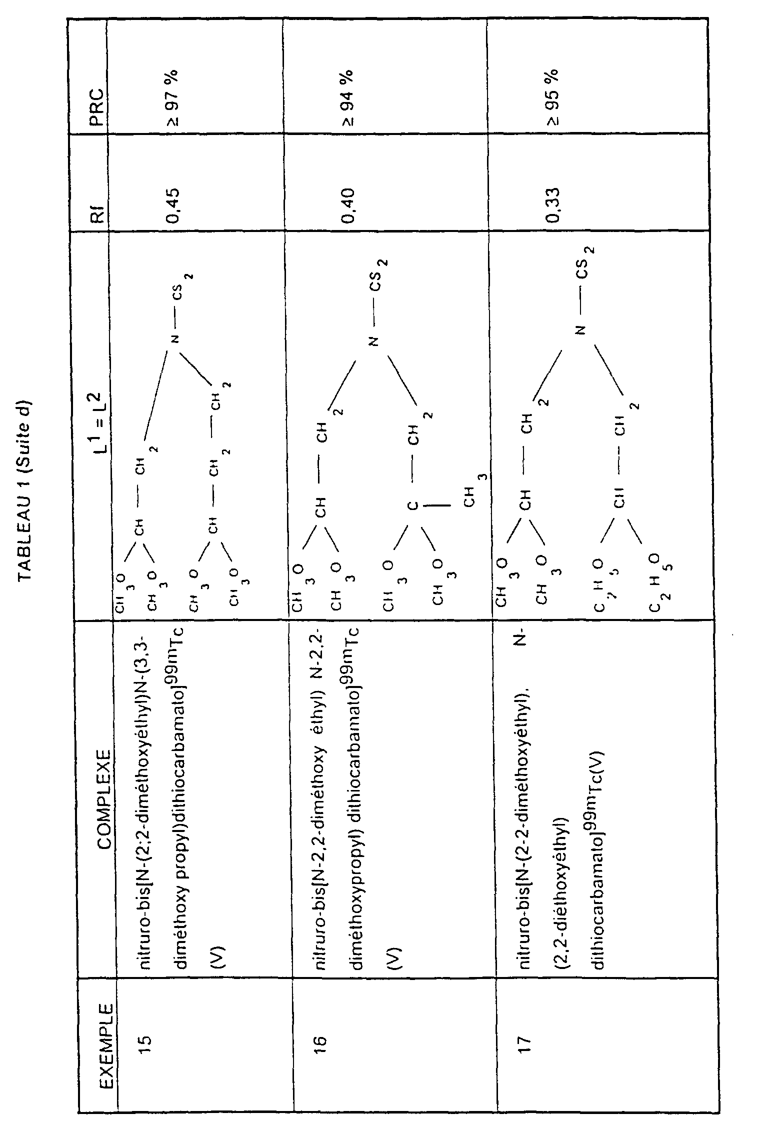

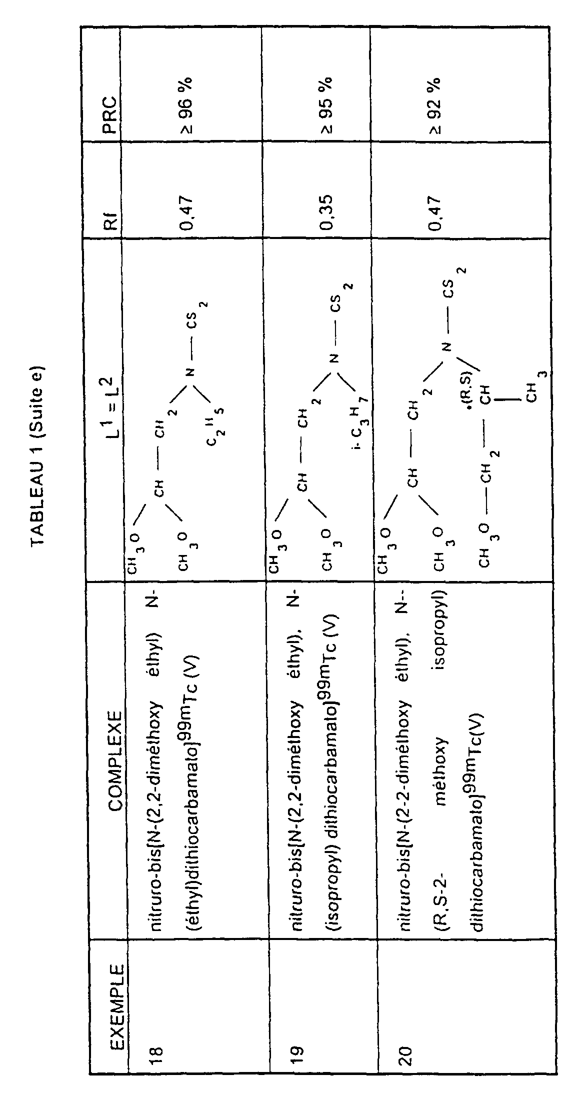

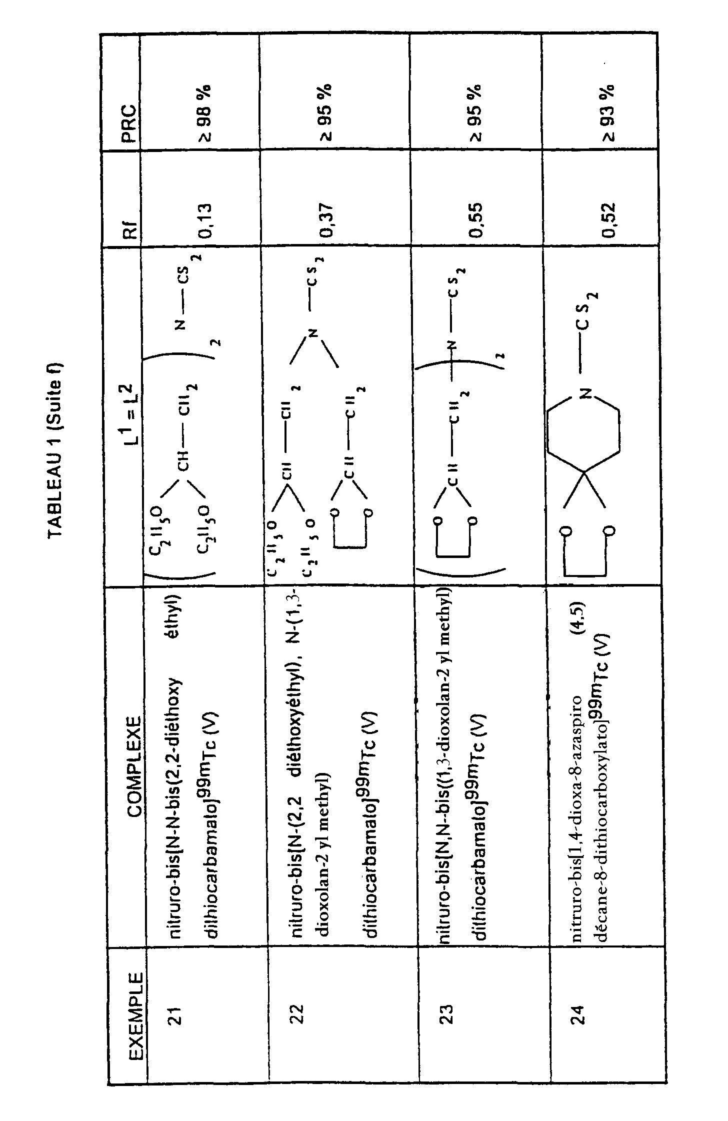

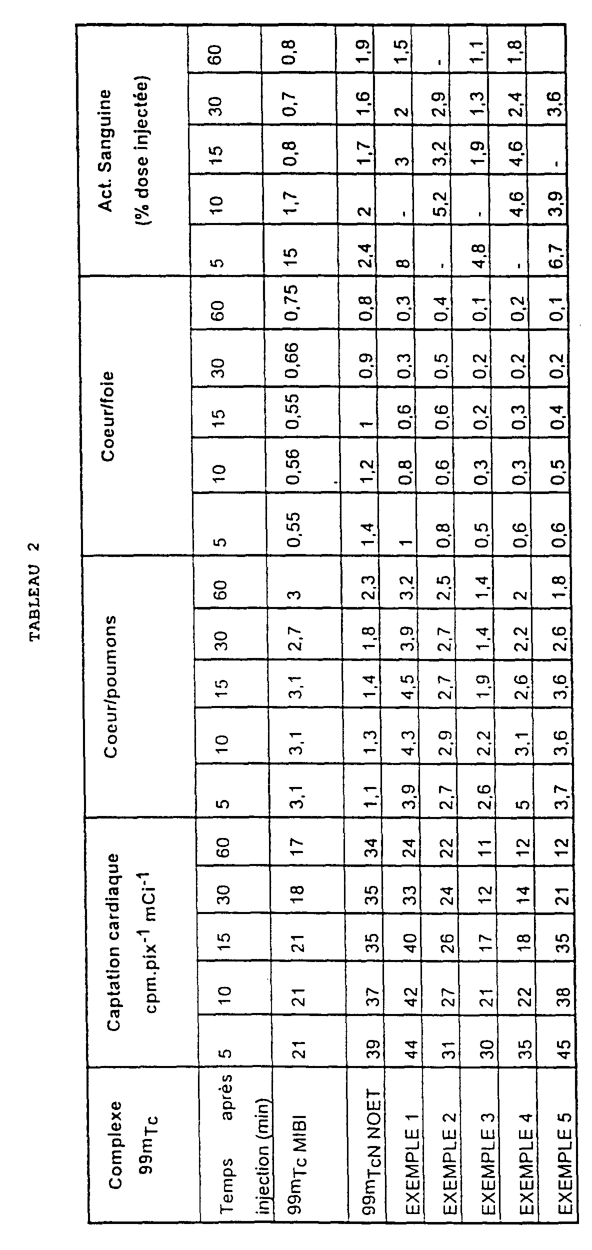

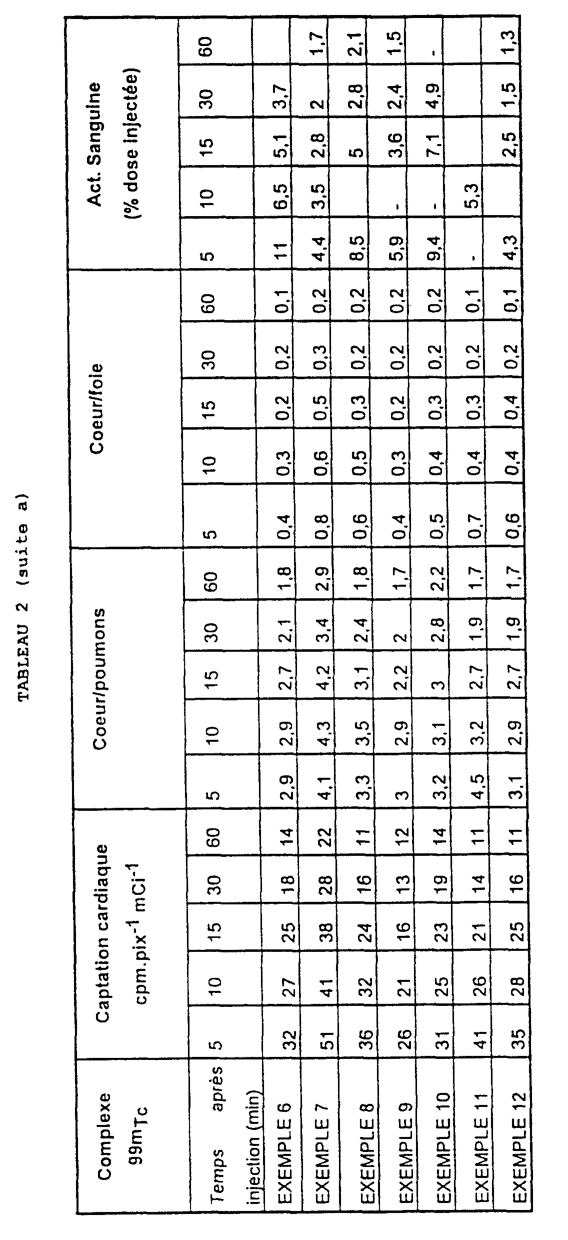

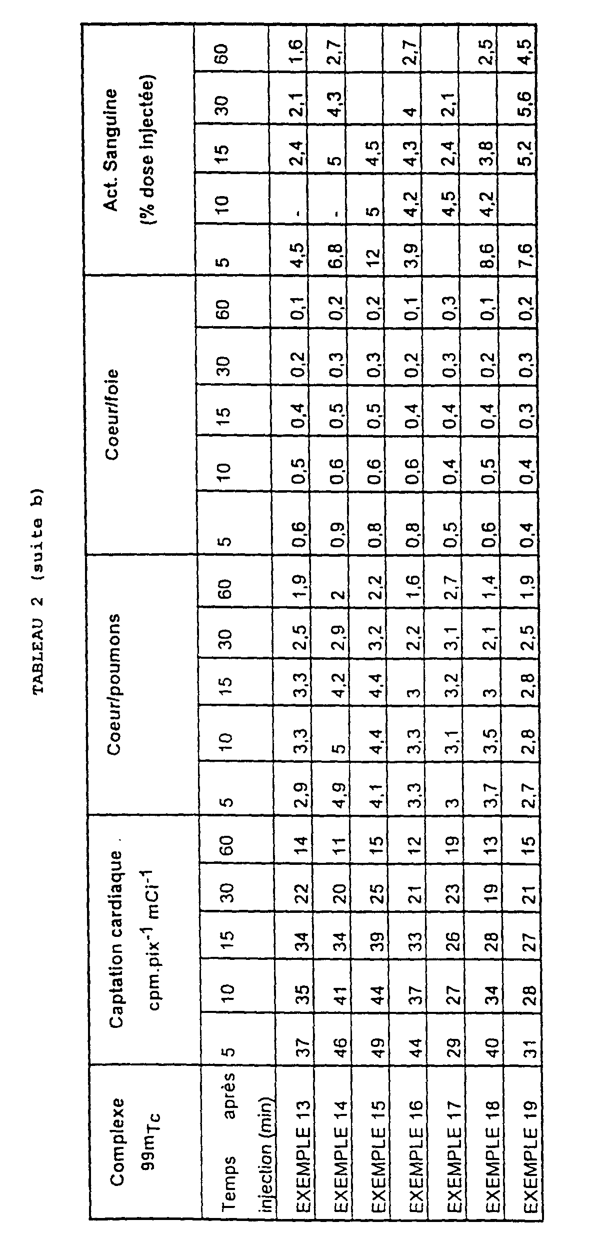

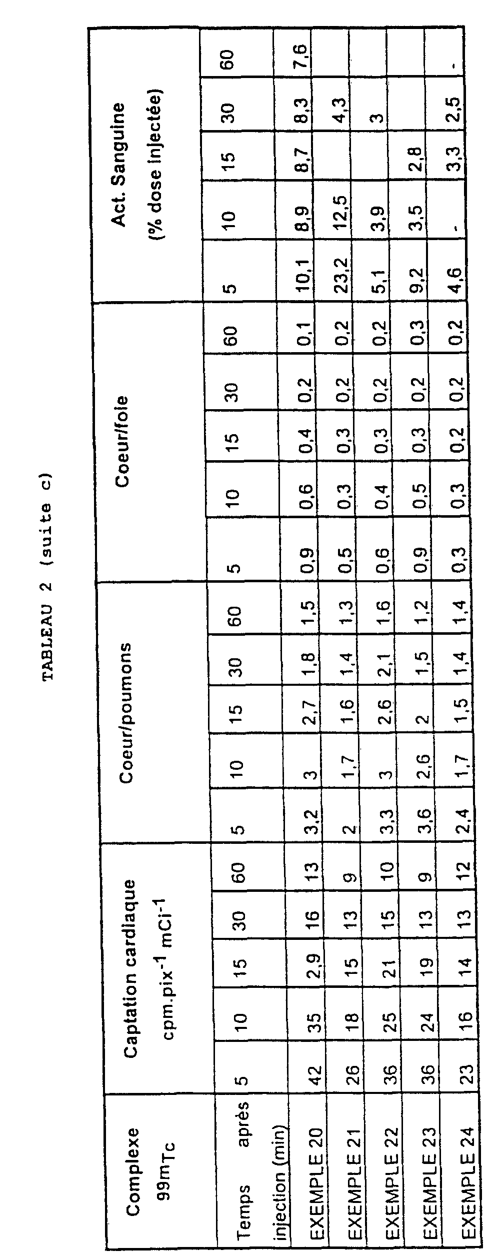

## Revendications

1. Produit radiopharmaceutique, **caractérisé en ce qu'**il comprend un complexe d'un métal de transition répondant à la formule :
(M ≡ N) L¹ L² (I)
dans laquelle M est un métal de transition, et L¹ et L² qui peuvent être identiques ou différents, répondent à la formule : 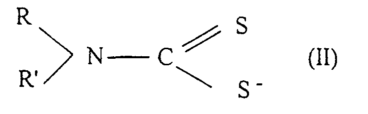 dans laquelle R et R' qui sont identiques ou différents, représentent :
- un groupe alkyle ;
- un groupe de formule : 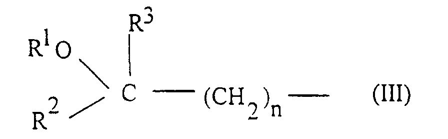 dans laquelle :
R¹ est un groupe alkyle, R² est un groupe alkyle ou un groupe alcoxy, ou R¹ et R² forment ensembe le groupe -CH₂CH₂0-,
R³ est un atome d'hydrogène ou un groupe alkyle, et n est égal à 0, 1, 2 ou 3 ;
- un groupe de formule : 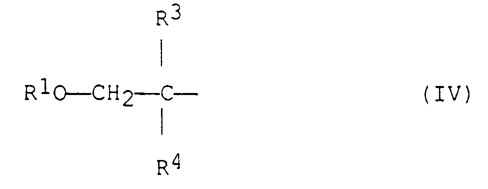 dans laquelle R¹ et R³ sont tels que définis ci-dessus et R⁴ est un groupe alkyle,
- le groupe de formule : 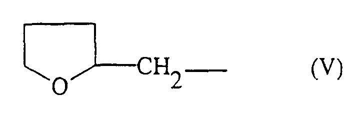
- un groupe de formule :
R¹O(CH₂CH₂O)ₚ CH₂CH₂- (VI)
dans laquelle R¹ est tel que défini ci-dessus, et p est égal à 1 ou 2, ou
dans laquelle R et R' forment ensemble avec l'atome d'azote auquel ils sont liés le groupe de formule : 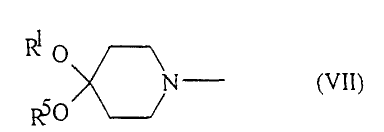 dans laquelle R¹ et R⁵ sont des groupes alkyle identiques ou différents, ou dans laquelle R¹ et R⁵ forment ensemble -CH₂-CH₂-, à condition que R et R' ne représentent pas tous deux un groupe alkyle.

2. Produit radiopharmaceutique selon la revendication 1, **caractérisé en ce que** M représente un isotope du technétium ou du rhénium.

3. Produit radiopharmaceutique selon la revendication 2, **caractérisé en ce que** l'isotope du technétium est Tc-99m.

4. Produit radiopharmaceutique selon la revendication 2, **caractérisé en ce que** l'isotope du rhénium est Re-186 ou Re-188.

5. Produit radiopharmaceutique selon l'une quelconque des revendications 1 à 4, **caractérisé en ce que** L¹ et L² sont identiques.

6. Produit radiopharmaceutique selon l'une quelconque des revendications 1 à 5, **caractérisé en ce que** l'un au moins des L¹ et L² répond à la formule (II)
dans laquelle :
- R et/ou R' représentent un groupe de formule : 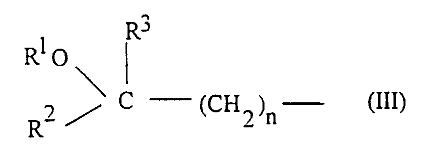
dans laquelle R¹ représente un groupe alkyle, R² représente un groupe alcoxy, R³ représente un atome d'hydrogène ou un groupe alkyle, et n est égal à 0, 1, 2 ou 3.

7. Produit radiopharmaceutique selon l'une quelconque des revendications 1 à 5, **caractérisé en ce que** l'un au moins des L¹ et L² répond à la formule (II)
dans laquelle :
- R et/ou R' représentent un groupe de formule : 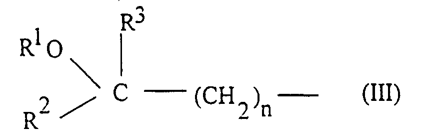
dans laquelle R¹, R² et R³ sont des groupes alkyle, identiques ou différents.

8. Produit radiopharmaceutique selon la revendication 1, **caractérisé en ce que** le complexe est choisi dans le groupe comprenant :
- le nitruro-bis[N-éthyl-N(R,S-2-méthoxy propyl) dithiocarbamato]^{99m}Tc (V),
- le nitruro-bis[N-éthyl-N(2-méthoxy isobutyl) dithiocarbamato] ^{99m}Tc(V),
- le nitruro-bis[N-,N-bis (2,2-diméthoxy éthyl) dithiocarbamato] ^{99m}Tc(V),
- le nitruro-bis[N-(2,2-diméthoxyéthyl), N-(3,3-diméthoxypropyl) dithiocarbamato] ^{99m}Tc(V),
- le nitruro-bis[N-(2,2-diméthoxyéthyl), N-(2,2 diméthoxy propyl) dithiocarbamato] ^{99m}Tc(V),
- le nitruro-bis[N-(2,2-diméthoxyéthyl), N-(2,2 diéthoxy éthyl) dithiocarbamato] ^{99m}Tc(V),
- le nitruro-bis-[N-(2,2-diméthoxyéthyl), N-éthyl dithiocarbamato] ^{99m}Tc(V),
- le nitruro-bis [N-(2,2-diméthoxyéthyl), N-isopropyl dithiocarbamato] ^{99m}Tc(V),
- le nitruro-bis[N,Nbis(2,2-diéthoxyéthyl), dithiocarbamato] ^{99m}Tc(V),
- le nitruro-bis[N-(2,2-diéthoxyéthyl), N-(1,3-dioxolan-2 yl méthyl) dithiocarbamato]^{99m}Tc (V), et
- le nitruro-bis-[N,N-bis (1,3-dioxolan-2 ylméthyl) dithiocarbamato] ^{99m}Tc(V).

9. Produit radiopharmaceutique selon l'une quelconque des revendications 1 à 5, **caractérisé en ce que** l'un au moins des L¹ et L² répond à la formule (II) dans laquelle :
- R et/ou R'répondent à la formule (IV): 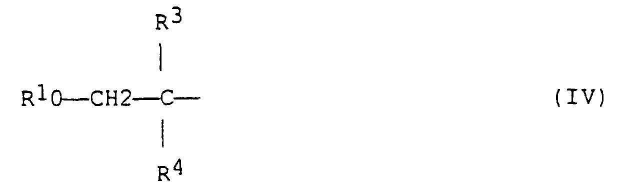
dans laquelle R¹ représente un groupe alkyle, R³ représente un atome d'hydrogéne et R⁴ représente un groupe alkyle

10. Produit radiopharmaceutique selon la revendication 9, **caractérisé en ce que** le complexe est choisi dans le groupe comprenant :
- le nitruro-bis[N-éthyl, N-((R,S)-2 méthoxy isopropyl)dithiocarbamato]^{99m}Tc (V),
- le nitruro-bis[ N-éthyl,N-(R-2-méthoxy isopropyl) dithiocarbamato]^{99m}Tc (V),
- le nitruro-bis[ N-éthyl, N(S-2-méthoxy isopropyl)dithiocarbamato]^{99m}Tc (V),
- le nitruro-bis [N-méthyl, N((R,S)-2- méthoxy isopropyl)dithiocarbamato] ^{99m}Tc(V),
- le nitruro-bis[ N-propyl, N-((R,S)2-méthoxy isopropyl)dithiocarbamato] ^{99m}Tc(V),
- le nitruro-bis[N-isopropyl, N((R,S)-2-méthoxy isopropyl) dithiocarbamato] ^{99m}Tc(V),
- le nitruro-bis[N-éthyl, N((R,S)-(1-méthoxy méthyl)propyl)dithiocarbamato] ^{99m}Tc(V),
- le nitruro-bis[N-éthyl, N-(2 méthoxy tert-butyl) dithiocarbamato] ^{99m}Tc(V), et
- le nitruro-bis[N-(2,2-diméthoxy éthyl), N((R,S)2-méthoxy isopropyl) dithiocarbamato] ^{99m}Tc(V).

11. Produit radiopharmaceutique selon l'une quelconque des revendications 1 à 5, **caractérisé en ce que** l'un au moins des L¹ et L² répond à la formule (II) dans laquelle :
- R et/ou R'représentent le groupe de formule :

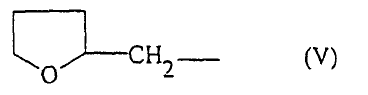

12. Produit radiopharmaceutique selon la revendication 11, **caractérisé en ce que** le complexe est choisi parmi :
- le nitruro-bis[ N-méthyl, N-((R,S) 1-tétrahydrofurfuryl)dithiocarbamato] ^{99m}Tc(V), et
- le nitruro-bis[N-éthyl, N-((R,S) 1-tétrahydrofurfuryl)dithiocarbamato]^{99m}Tc (V).

13. Produit radiopharmaceutique selon la revendication 1, **caractérisé en ce que** le complexe est le nitruro-bis[N-éthyl, N-(2(2-méthoxy éthoxy) éthyl) dithiocarbamato] ^{99m}Tc(V)ou le nitruro-bis [1,4 -dioxa-8-azaspiro (4,5) décane-8-dithiocarboxylato] ^{99m}Tc (V).

14. Procédé de préparation d'un produit radiopharmaceutique selon la revendication 1, **caractérisé en ce qu'**il consiste à faire réagir en solution un composé oxygéné du métal de transition M, avec
- 1) un ligand azoté constitué soit par un azoture d'ammonium ou de métal pharmaceutiquement acceptable, soit par un composé azoté comportant un motif N-N dans lequel les N sont reliés à des atomes d'hydrogéne et/ou à des groupements organiques monovalents, par exemple par l'intermédiaire d'un atome de carbone ou d'un atome de S, ou dans lequel l'un des N est relié à l'atome de carbone d'un groupement organique bivalent par l'intermédiaire d'une double liaison et l'autre N est relié à des atomes d'hydrogéne et/ou à des groupements organiques monovalents, par exemple par l'intermédiaire d'un atome de carbone ;
- 2) un agent réducteur constitué soit par du dithionite d'ammonium ou de métal pharmaceutiquement acceptable, soit par de l'étain II présent sous forme ionique dans la solution, et
- 3) un composé répondant à la formule : 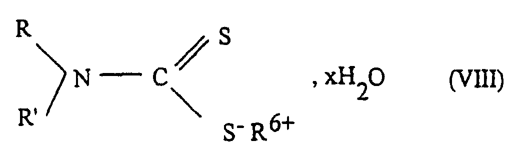
dans laquelle R et R' ont les significations données ci-dessus, R⁶ est un ion de métal alcalin, H⁺ ou NH₄⁺, et x est égal à 0 ou est un nombre entier allant de 1 à 5.

15. Procédé selon la revendication 14, **caractérisé en ce que** le ligand azoté est le succinyl dihydrazide et l'agent réducteur est le chlorure stanneux dihydraté.

16. Trousse pour la préparation d'un produit radiopharmaceutique selon la revendication 1, **caractérisé en ce qu'**elle comprend
- un premier flacon contenant un sel d'étain et un agent complexant capable de maintenir l'étain sous forme ionique,
- un deuxième flacon contenant un ligand azoté, et
- un troisième flacon contenant un composé répondant à la formule : 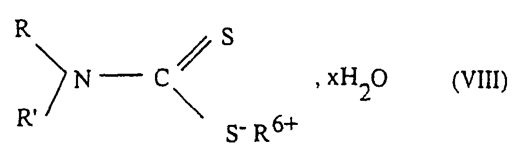
dans laquelle R et R' ont les significations données dans la revendication 1, R⁶ est un ion de métal alcalin, H⁺ ou NH₄⁺, et x est égal à 0 ou est un nombre entier de 1 à 5.

17. Trousse pour la préparation d'un produit radiopharmaceutique selon la revendication 1, **caractérisée en ce qu'**elle comprend :
- un premier flacon contenant un sel d'étain, un ligand azoté et un agent complexant capable de maintenir l'étain sous forme ionique, et
- un deuxième flacon contenant un composé de formule : 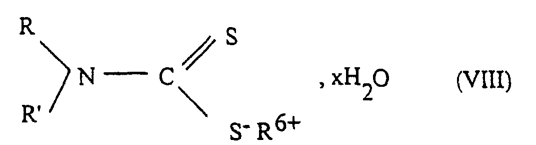
dans laquelle R et R' ont les significations données dans la revendication 1, R⁶ est un ion de métal alcalin, H⁺ ou NH₄⁺, et x est égal à 0 ou est un nombre entier de 1 à 5.

18. Trousse selon la revendication 16 ou 17, **caractérisée en ce que** le composé de formule (VIII) est choisi parmi :
- le N-éthyl, N-((R,S)-2-méthoxy isopropyl) dithiocarbamate de sodium,
- le N-éthyl, N((R)-2-méthoxy isopropyl) dithiocarbamate de sodium,
- le N-éthyl, N-((S)-2 méthoxy isopropyl) dithiocarbamate de sodium,
- le N,N-bis(2,2-diméthoxy éthyl) dithiocarbamate de sodium,
- le N-(2,2-diméthoxyéthyl), N-(3,3-diméthoxypropyl) dithiocarbamate de sodium,
- le N-(2,2-diméthoxyéthyl), N-éthyl dithiocarbamate de sodium.

19. Trousse selon l'une quelconque des revendications 16 à 18, **caractérisé en ce que** le sel d'étain est le chlorure stanneux, l'agent complexant est l'acide 1,2-diaminopropane - N, N, N', N'-tétracétique ou un sel de celui-ci, et le ligand azoté est le succinyl dihydrazide.

20. Produit radiopharmaceutique selon l'une quelconque des revendications 1 à 13 pour la scintigraphie du myocarde.

## Patentansprüche

1. Radiopharmazeutisches Produkt, **dadurch gekennzeichnet, daß** es einen Übergangsmetallkomplex der Formel:
(M ≡ N) L¹ L² (I)
worin M für ein Übergangsmetall steht und L¹ und L², die gleich oder verschieden sein können, der Formel: 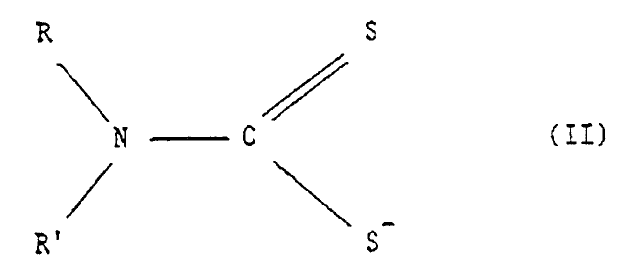 entsprechen, worin R und R', die gleich oder verschieden sind, für:
- eine Alkylgruppe;
- eine Gruppe der Formel: 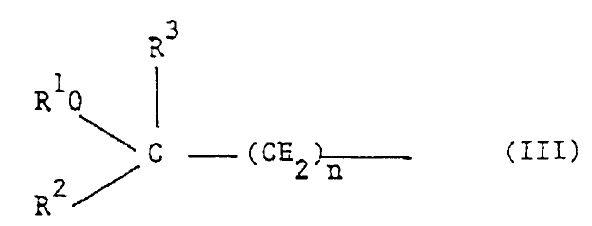 worin:
R¹ für eine Alkylgruppe und R² für eine Alkylgruppe oder eine Alkoxygruppe steht oder R¹ und R² gemeinsam die Gruppe -CH₂CH₂O- bilden,
R³ für ein Wasserstoffatom oder eine Alkylgruppe steht und n gleich 0, 1, 2 oder 3 ist;
- eine Gruppe der Formel: 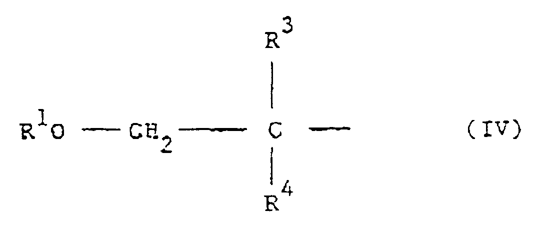 worin R¹ und R³ die oben angegebene Bedeutung besitzen und R⁴ für eine Alkylgruppe steht;
- die Gruppe der Formel: 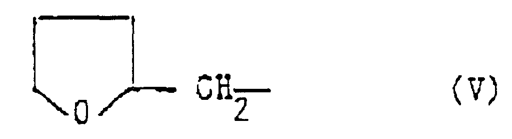
- eine Gruppe der Formel:
R¹O(CH₂CH₂O)ₚCH₂CH₂- (VI)
worin R¹ die oben angegebene Bedeutung besitzt und p gleich 1 oder 2 ist, stehen oder
worin R und R' gemeinsam mit dem Stickstoffatom, an das sie gebunden sind, die Gruppe der Formel: 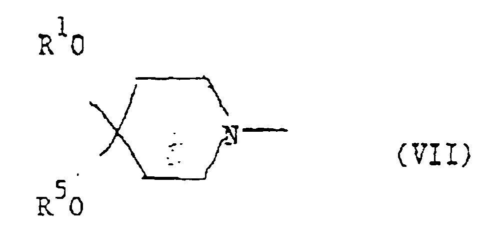 worin R¹ und R⁵ für gleiche oder verschiedene Alkylgruppen stehen oder R¹ und R⁵ gemeinsam für -CH₂-CH₂- stehen, bilden mit der Maßgabe, daß R und R' nicht gleichzeitig für eine Alkylgruppe stehen, enthält.

2. Radiopharmazeutisches Produkt nach Anspruch 1, **dadurch gekennzeichnet, daß** M ein Isotop des Technetiums oder des Rheniums darstellt.

3. Radiopharmazeutisches Produkt nach Anspruch 2, **dadurch gekennzeichnet, daß** es sich bei dem Technetiumisotop um Tc-99m handelt.

4. Radiopharmazeutisches Produkt nach Anspruch 2, **dadurch gekennzeichnet, daß** es sich bei dem Rheniumisotop um Re-186 oder Re-188 handelt.

5. Radiopharmazeutisches Produkt nach einem der Ansprüche 1 bis 4, **dadurch gekennzeichnet, daß** L¹ und L² gleich sind.

6. Radiopharmazeutisches Produkt nach einem der Ansprüche 1 bis 5, **dadurch gekennzeichnet, daß** mindestens eine der Gruppen L¹ und L² der Formel (II) entspricht, worin:
- R und/oder R' für eine Gruppe der Formel: 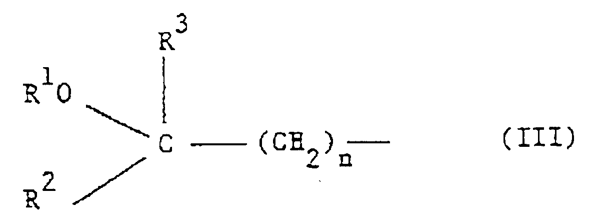
worin R¹ für eine Alkylgruppe steht, R² für eine Alkoxygruppe steht, R³ für ein Wasserstoffatom oder eine Alkylgruppe steht und n gleich 0, 1, 2 oder 3 ist, stehen.

7. Radiopharmazeutisches Produkt nach einem der Ansprüche 1 bis 5, **dadurch gekennzeichnet, daß** mindestens eine der Gruppen L¹ und L² der Formel (II) entspricht, worin:
- R und/oder R' für eine Gruppe der Formel: 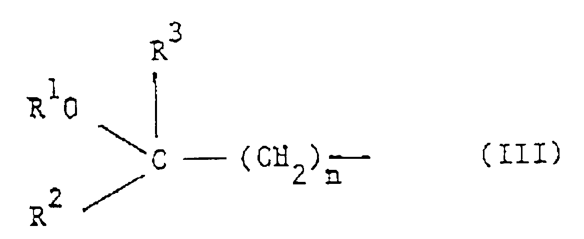
worin R¹, R² und R³ für gleiche oder verschiedene Alkylgruppen stehen, stehen.

8. Radiopharmazeutisches Produkt nach Anspruch 1, **dadurch gekennzeichnet, daß** der Komplex aus der Gruppe bestehend aus:
- Nitrido-bis[N-ethyl-N-(R,S-2-methoxypropyl)-dithiocarbamato] ^{99m}Tc(V),
- Nitrido-bis[N-ethyl-N-(2-methoxyisobutyl)-dithiocarbamato] ^{99m}Tc(V),
- Nitrido-bis[N,N-bis(2,2-dimethoxyethyl)-dithiocarbamato] ^{99m}Tc(V),
- Nitrido-bis[N-(2,2-dimethoxyethyl)-N-(3,3-dimethoxypropyl)-dithiocarbamato] ^{99m}Tc(V),
- Nitrido-bis[N-(2,2-dimethoxyethyl)-N-(2,2-dimethoxypropyl)-dithiocarbamato] ^{99m}Tc(V),
- Nitrido-bis[N-(2,2-dimethoxyethyl)-N-(2,2-diethoxyethyl)-dithiocarbamato] ^{99m}Tc(V),
- Nitrido-bis[N-(2,2-dimethoxyethyl)-N-ethyldithiocarbamato] ^{99m}Tc(V),
- Nitrido-bis[N-(2,2-dimethoxyethyl)-N-isopropyldithiocarbamato] ^{99m}Tc(V),
- Nitrido-bis[N,N-bis(2,2-diethoxyethyl)-dithiocarbamato] ^{99m}Tc(V),
- Nitrido-bis[N-(2,2-diethoxyethyl)-N-(1,3-dioxolan-2-ylmethyl)-dithiocarbamato] ^{99m}Tc(V) und
- Nitrido-bis[N,N-bis(1,3-dioxolan-2-ylmethyl)-dithiocarbamato] ^{99m}Tc(V)
ausgewählt ist.

9. Radiopharmazeutisches Produkt nach einem der Ansprüche 1 bis 5, **dadurch gekennzeichnet, daß** mindestens eine der Gruppen L¹ und L² der Formel (II) entspricht, worin:
- R und/oder R' der Formel (IV): 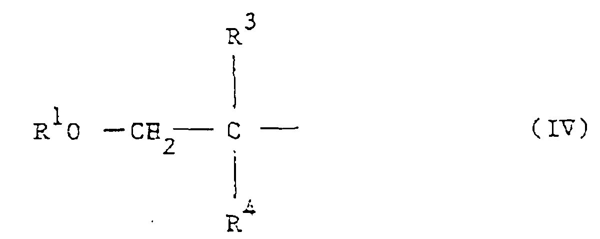
worin R¹ für eine Alkylgruppe steht, R³ für ein Wasserstoffatom steht und R⁴ für eine Alkylgruppe steht, entsprechen.

10. Radiopharmazeutisches Produkt nach Anspruch 9,
**dadurch gekennzeichnet, daß** der Komplex aus der Gruppe bestehend aus:
- Nitrido-bis[N-ethyl-N-((R,S)-2-methoxyisopropyl)dithiocarbamato]^{99m}Tc (V),
- Nitrido-bis[N-ethyl-N-(R-2-methoxyisopropyl)-dithiocarbamato]^{99m}Tc(V),
- Nitrido-bis[N-ethyl-N-(S-2-methoxyisopropyl)-dithiocarbamato]^{99m}Tc (V),
- Nitrido-bis[N-methyl-N-((R,S)-2-methoxyisopropyl)dithiocarbamato] ^{99m}Tc(V)
- Nitrido-bis[N-propyl-N-((R,S)-2-methoxyisopropyl) dithiocarbamato] ^{99m}Tc(V),
- Nitrido-bis[N-isopropyl-N-((R,S)-2-methoxyisopropyl)-dithiocarbamato]^{99m}Tc (V),
- Nitrido-bis[N-ethyl-N-((R,S)-1-(methoxymethyl)-propyl)-dithiocarbamato]^{99m}Tc (V),
- Nitrido-bis[N-ethyl-N-(2-methoxy-tert.-butyl)-dithiocarbamato] ^{99m}TC(V) und
- Nitrido-bis[N-(2,2-dimethoxyethyl)-N-(R,S-2-methoxyisopropyl)-dithiocarbamato]^{99m}Tc (V) ausgewählt ist.

11. Radiopharmazeutisches Produkt nach einem der Ansprüche 1 bis 5, **dadurch gekennzeichnet, daß** mindestens eine der Gruppen L¹ und L² der Formel (II) entspricht, worin:
- R und/oder R' für die Gruppe der Formel: 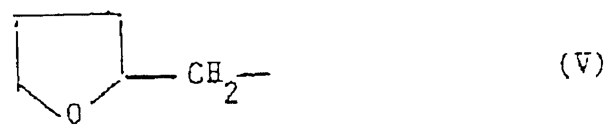 stehen.

12. Radiopharmazeutisches Produkt nach Anspruch 11, **dadurch gekennzeichnet, daß** der Komplex unter:
- Nitrido-bis[N-methyl-N-(R,S-1-tetrahydrofurfuryl)dithiocarbamato]^{99m}Tc (V) und
- Nitrido-bis[N-ethyl-N-(R,S-1-tetrahydrofurfuryl)dithiocarbamato] ^{99m}Tc(V)
ausgewählt ist.

13. Radiopharmazeutisches Produkt nach Anspruch 1,
**dadurch gekennzeichnet, daß** es sich bei dem Komplex um Nitrido-bis[N-ethyl-N-(2-(2-methoxyethoxy)ethyl)-dithiocarbamato] ^{99m}Tc(V) oder Nitrido-bis[1,4-dioxa-8-azaspiro(4,5)decan-8-dithio-carboxylato] ^{99m}Tc(V) handelt.

14. Verfahren zur Herstellung eines radiopharmazeutischen Produkts nach Anspruch 1, **dadurch gekennzeichnet, daß** man eine Sauerstoffverbindung des Übergangsmetalls M in Lösung mit
- 1) einem Stickstoffliganden, bei dem es sich entweder um Ammoniumazid oder ein Azid eines pharmazeutisch unbedenklichen Metalls oder eine Stickstoffverbindung mit einer N-N-Einheit, in welcher. die N-Atome an Wasserstoffatome und/oder an einwertige organische Gruppen gebunden sind, beispielsweise über ein Kohlenstoffatom oder ein S-Atom, oder in welcher das eine N-Atom über eine Doppelbindung an das Kohlenstoffatom einer zweiwertigen organischen Gruppe gebunden ist und das andere N-Atom an Wasserstoffatome und/oder an einwertige organische Gruppen gebunden ist, beispielsweise über ein Kohlenstoffatom, handelt;
- 2) einem Reduktionsmittel, bei dem es sich entweder um Ammoniumdithionit oder das Dithionit eines pharmazeutisch unbedenklichen Metalls oder in der Lösung in Ionenform vorhandenes Zinn(II) handelt, und
- 3) einer Verbindung der Formel: 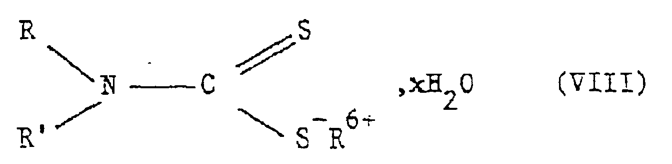 worin R und R' die oben angegebene Bedeutung besitzen, R⁶ für ein Alkalimetallion, H⁺ oder NH₄⁺ steht und x für 0 oder eine ganze Zahl von 1 bis 5 steht,
umsetzt.

15. Verfahren nach Anspruch 14, **dadurch gekennzeichnet, daß** man als Stickstoffliganden Bernsteinsäuredihydrazid und als Reduktionsmittel Zinn(II)-chloriddihydrat einsetzt.

16. Kit für die Herstellung eines radiopharmazeutischen Produkts nach Anspruch 1, **dadurch gekennzeichnet, daß** es
- eine erste Flasche, die ein Zinnsalz und einen Komplexbildner, der das Zinn in Ionenform halten kann, enthält,
- eine zweite Flasche, die einen Stickstoffliganden enthält, und
- eine dritte Flasche, die eine Verbindung der Formel: 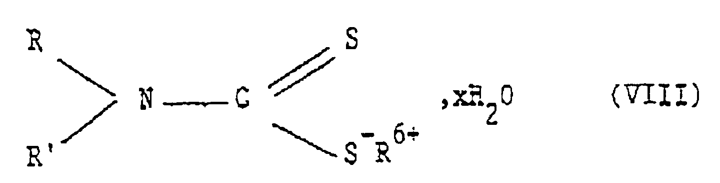
worin R und R' die in Anspruch 1 angegebene Bedeutung besitzen, R⁶ für ein Alkalimetallion, H⁺ oder NH₄⁺ steht und x für 0 oder eine ganze Zahl von 1 bis 5 steht, enthält, umfaßt.

17. Kit für die Herstellung eines radiopharmazeutischen Produkts nach Anspruch 1, **dadurch gekennzeichnet, daß** es
- eine erste Flasche, die ein Zinnsalz, einen Stickstoffliganden und einen Komplexbildner, der das Zinn in Ionenform halten kann, enthält, und
- eine zweite Flasche, die eine Verbindung der Formel: 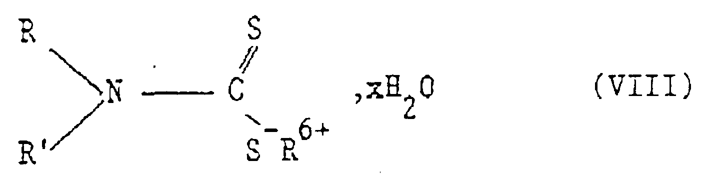
worin R und R' die in Anspruch 1 angegebene Bedeutung besitzen, R⁶ für ein Alkalimetallion, H⁺ oder NH₄⁺ steht und x für 0 oder eine ganze Zahl von 1 bis 5 steht, enthält,
umfaßt.

18. Kit nach Anspruch 16 oder 17, **dadurch gekennzeichnet, daß** die Verbindung der Formel (VIII) unter:
- Natrium-N-ethyl-N-((R,S)-2-methoxyisopropyl)-dithiocarbamat,
- Natrium-N-ethyl-N-((R)-2-methoxyisopropyl)-dithiocarbamat,
- Natrium-N-ethyl-N-((S)-2-methoxyisopropyl)-dithiocarbamat,
- Natrium-N,N-bis(2,2-dimethoxyethyl)-dithiocarbamat,
- Natrium-N-(2,2-dimethoxyethyl)-N-(3,3-dimethoxypropyl)-dithiocarbamat und
- Natrium-N-(2,2-dimethoxyethyl)-N-ethyl-dithiocarbamat
ausgewählt ist.

19. Kit nach einem der Ansprüche 16 bis 18, **dadurch gekennzeichnet, daß** es sich bei dem Zinnsalz um Zinn(II)-chlorid, bei dem Komplexbildner um 1,2-Diaminopropan-N,N,N',N'-tetraessigsäure oder ein Salz davon und bei dem Stickstoffliganden um Bernsteinsäuredihydrazid handelt.

20. Radiopharmazeutisches Produkt nach einem der Ansprüche 1 bis 13 für die Myokardszintigraphie.

## Claims

1. Radiopharmaceutical product, **characterized in that** it comprises a complex of a transition metal complying with the formula:
(M≡N)L¹L² (I)
in which M is a transition metal, and L¹ and L², which can be the same or different, comply with the formula: 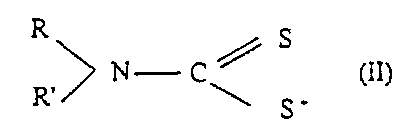 in which R and R', which are the same or different, represent:
- an alkyl group;
- a group of formula: 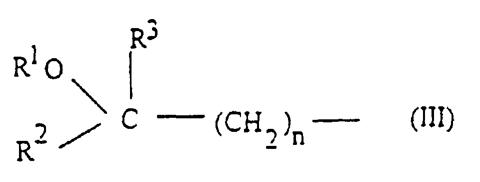 in which:
R¹ is an alkyl group, R² is an alkyl group or an alkoxy group, or R¹ and R² form together the group -CH₂CH₂O-,
R³ is a hydrogen atom or an alkyl group and n is equal to 0, 1, 2 or 3;
- a group of formula: 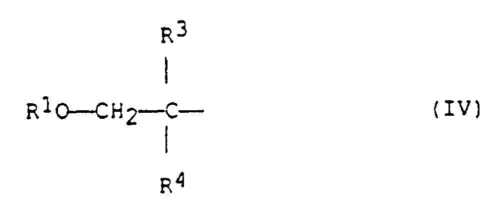 in which R¹ and R³ are as defined hereinbefore and R⁴ is an alkyl group,
- the group of formula: 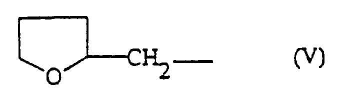
- a group of formula:
R¹O(CH₂CH₂O)ₚCH₂CH₂- (VI)
in which R¹ is as defined hereinbefore and p is equal to 1 or 2, or
in which R and R' together with the nitrogen atom to which they are linked form the group of formula: 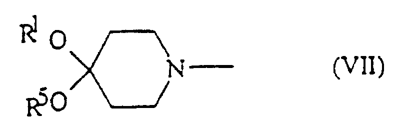 in which R¹ and R⁵ are identical or different alkyl groups, or in which R¹ and R⁵ form together -CH₂-CH₂-, provided that both R and R' do not represent an alkyl group.

2. Radiopharmaceutical product according to Claim 1, **characterized in that** M represents an isotope of technetium or rhenium.

3. Radiopharmaceutical product according to Claim 2, **characterized in that** the isotope of technetium is Tc-99m.

4. Radiopharmaceutical product according to Claim 2, **characterized in that** the isotope of rhenium is Re-186 or Re-188.

5. Radiopharmaceutical product according to any one of Claims 1 to 4, **characterized in that** L¹ and L² are identical.

6. Radiopharmaceutical product according to any one of Claims 1 to 5, **characterized in that** at least one of L¹ and L² complies with the formula (II) in which:
- R and/or R' represent a group of formula: 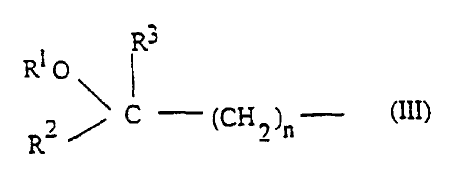 in which R¹ represents an alkyl group, R² represents an alkoxy group, R³ represents a hydrogen atom or an alkyl group, and n is equal to 0, 1, 2 or 3.

7. Radiopharmaceutical product according to any one of Claims 1 to 5, **characterized in that** at least one of L¹ and L² complies with the formula (II) in which:
- R and/or R' represent a group of formula: 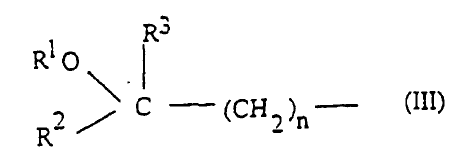 in which R¹, R² and R³ are identical or different alkyl groups.

8. Radiopharmaceutical product according to Claim 1, **characterized in that** the complex is chosen within the group comprising:
- nitridobis[N-ethyl-N-(R,S-2-methoxypropyl)dithiocarbamato]^{99m}Tc (V),
- nitridobis[N-ethyl-N-(2-methoxyisobutyl)dithiocarbamato]^{99m}Tc (V),
- nitridobis[N,N-bis(2,2-dimethoxyethyl)dithiocarbamato]^{99m}TC (V),
- nitridobis[N-(2,2-dimethoxyethyl)-N-(3,3-dimethoxypropyl)dithiocarbamato]^{99m}Tc (V) ,
- nitridobis[N-(2,2-dimethoxyethyl)-N-(2,2-dimethoxypropyl)dithiocarbamato] ^{99m}Tc(V),
- nitridobis[N-(2,2-dimethoxyethyl)-N-(2,2-diethoxyethyl)dithiocarbamato]^{99m}Tc (V),
- nitridobis[N-(2,2-dimethoxyethyl)-N-ethyldithiocarbamato] ^{99m}Tc(V),
- nitridobis[N-(2,2-dimethoxyethyl)-N-isopropyldithiocarbamato] ^{99m}Tc (V),
- nitridobis[N,N-bis(2,2-diethoxyethyl)dithiocarbamato] ^{99m}Tc(V),
- nitridobis[N-(2,2-diethoxyethyl)-N-(1,3-dioxolan-2-ylmethyl)dithiocarbamato] ^{99m}Tc(V),
and
- nitridobis[N,N-bis(1,3-dioxolan-2-ylmethyl)dithiocarbamato] ^{99m}Tc(V).

9. Radiopharmaceutical product according to any one of Claims 1 to 5, **characterized in that** at least one of L¹ and L² complies with the formula (II) in which:
- R and/or R' comply with the formula (IV): 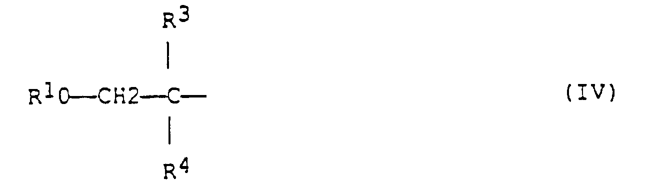 in which R¹ represents an alkyl group, R³ represents a hydrogen atom and R⁴ represents an alkyl group.

10. Radiopharmaceutical product according to Claim 9, **characterized in that** the complex is chosen within the group comprising:
- nitridobis[N-ethyl-N-((R,S)-2-methoxyisopropyl)dithiocarbamato] ^{99m}Tc(V),
- nitridobis[N-ethyl-N-(R-2-methoxyisopropyl)-dithiocarbamato] ^{99m}Tc(V),
- nitridobis[N-ethyl-N-(S-2-methoxyisopropyl)-dithiocarbamato] ^{99m}Tc(V),
- nitridobis[N-methyl-N-((R,S)-2-methoxyisopropyl) dithiocarbamato] ^{99m}Tc (V),
- nitridobis[N-propyl-N-((R,S)-2-methoxyisopropyl) dithiocarbamato] ^{99m}Tc (V),
- nitridobis[N-isopropyl-N-((R,S)-2-methoxyisopropyl)dithiocarbamato] ^{99m}Tc(V),
- nitridobis[N-ethyl-N-((R,S)-(1-methoxymethyl)-propyl) dithiocarbamato] ^{99m}Tc (V),
- nitridobis[N-ethyl-N-(2-methoxy-tert-butyl)-dithiocarbamato] ^{99m}Tc(V), and
- nitridobis[N-(2,2-dimethoxyethyl)-N-((R,S)-2-methoxyisopropyl)dithiocarbamato] ^{99m}Tc(V).

11. Radiopharmaceutical product according to any one of Claims 1 to 5, **characterized in that** at least one of L¹ and L² complies with the formula (II) in which:
- R and/or R' represent the group of formula:

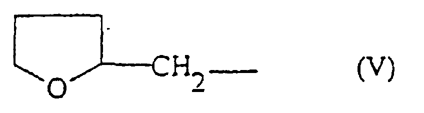

12. Radiopharmaceutical product according to Claim 11, **characterized in that** the complex is chosen from among:
- nitridobis[N-methyl-N-((R,S)-1-tetrahydrofurfuryl)dithiocarbamato] ^{99m}Tc(V), and
- nitridobis [N-ethyl-N-((R,S)-1-tetrahydrofurfuryl)dithiocarbamato]^{99m}Tc (V).

13. Radiopharmaceutical product according to Claim 1, **characterized in that** the complex is nitridobis[N-ethyl-N-(2-(2-methoxyethoxy)ethyl)dithiocarbamato] ^{99m}Tc(V) or nitridobis [1,4-dioxa-8-azaspiro[4.5]decane-8-dithiocarboxylato] ^{99m}Tc(V).

14. Process for the preparation of a radiopharmaceutical product according to Claim 1, **characterized in that** it consists in reacting, in solution, an oxygen compound of the transition metal M with
- 1) a nitrogen ligand composed either of a pharmaceutically acceptable metal or ammonium azide, or of a nitrogen compound having an N-N unit in which the Ns are connected to hydrogen atoms and/or to monovalent organic groups, e.g. via a carbon atom or an S atom, or in which one of the Ns is connected to the carbon atom of a divalent organic group via a double bond and the other N is connected to hydrogen atoms and/or to monovalent organic groups, e.g. via a carbon atom;
- 2) a reducing agent composed either of pharmaceutically acceptable metal or ammonium dithionite, or of tin(II) present in the ionic form in the solution, and
- 3) a compound complying with the formula: 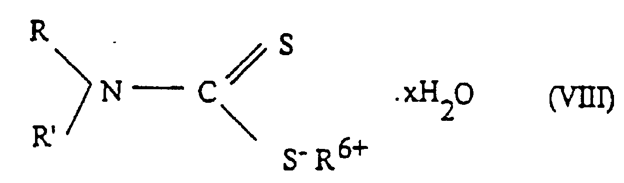 in which R and R' have the meanings given hereinbefore, R⁶ is an alkali metal, H⁺ or NH₄⁺ ion, and x is equal to 0 or is an integer from 1 to 5.

15. Process according to Claim 14, **characterized in that** the nitrogen ligand is succinyl dihydrazide and the reducing agent is stannous chloride dihydrate.

16. Kit for the preparation of a radiopharmaceutical product according to Claim 1, **characterized in that** it comprises:
- a first bottle containing a tin salt and a complexing agent able to keep the tin in the ionic form,
- a second bottle containing a nitrogen ligand, and
- a third bottle containing a compound complying with the formula: 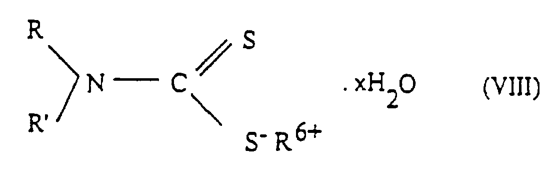 in which R and R' have the meanings given in Claim 1, R⁶ is an alkali metal, H⁺ or NH₄⁺ ion, and x is equal to 0 or is an integer from 1 to 5.

17. Kit for the preparation of a radiopharmaceutical product according to Claim 1, **characterized in that** it comprises:
- a first bottle containing a tin salt, a nitrogen ligand and a complexing agent able to keep the tin in the ionic form, and
- a second bottle containing a compound of formula: 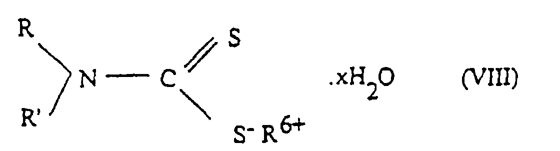 in which R and R' have the meanings given in Claim 1, R⁶ is an alkali metal, H⁺ or NH₄⁺ ion, and x is equal to 0 or is an integer from 1 to 5.

18. Kit according to Claim 16 or 17, **characterized in that** the compound of formula (VIII) is chosen from among:
- sodium N-ethyl-N-((R,S)-2-methoxyisopropyl)dithiocarbamate,
- sodium N-ethyl-N-((R)-2-methoxyisopropyl)dithiocarbamate,
- sodium N-ethyl-N-((S)-2-methoxyisopropyl)dithiocarbamate,
- sodium N,N-bis(2,2-dimethoxyethyl)dithiocarbamate,
- sodium N-(2,2-dimethoxyethyl)-N-(3,3-dimethoxypropyl)dithiocarbamate,
- sodium N-(2,2-dimethoxyethyl)-N-ethyldithiocarbamate.

19. Kit according to any one of Claims 16 to 18, **characterized in that** the tin salt is stannous chloride, the complexing agent is 1,2-diaminopropane-N,N,N',N'-tetraacetic acid or a salt thereof, and the nitrogen ligand is succinyl dihydrazide.

20. Radiopharmaceutical product according to any one of Claims 1 to 13 for scintigraphy of the myocardium.
